# FASCICULE DE BREVET EUROPEEN

(11) **EP 0 399 856 B1**
(45) Date de publication et mention de la délivrance du brevet: **09.08.1995**
(21) Numéro de dépôt: 90400827.3
(22) Date de dépôt: 27.03.1990
(51) Int. Cl.: C07D 475/02, C07D 475/06, C07D 239/48

(54) **Pteridin-4 (3H)-ones, procédés de préparation et médicaments les contenant**
Pteridin-4 (3H)-onen, Verfahren zu ihrer Herstellung und sie enthaltende Medikamente
Pteridin-4 (3H)-ones, processes for their preparation and medicaments containing them

(30) Priorité: 30.03.1989 FR 8904193
(43) Date de publication de la demande: 28.11.1990
(73) Titulaire: LIPHA, LYONNAISE INDUSTRIELLE PHARMACEUTIQUE, F-69359 Lyon Cedex 08 (FR)
(72) Inventeur: Ferrand, Gérard, F-69005 Lyon (FR); Dumas, Hervé, F-38090 Villefontaine (FR); Depin, Jean-Claude, F-69003 Lyon (FR); Quentin, Yvette, F-69004 Lyon (FR)
(74) Mandataire: Polus, Camille

(56) Documents cités:
- FR-A- 2 190 447
- US-A- 3 819 631
- JOURNAL OF MEDICINAL CHEMISTRY, vol. 15, no. 2, 1972, pages 210-211, American Chemical Society, Washington, DC, US; E. FELDER et al.: "Synthesis of 4(3H)- pteridinones"

## Description

La présente invention concerne des ptéridin-4(3H)-ones, des procédés permettant de les préparer et leur application dans le domaine thérapeutique.

Le rôle biologique des ptéridin-4(3H)-ones est illustré par les 2-alcoylptéridin-4(3H)-ones décrites par V. Liede et al. dans le brevet allemand 2.232.098 et revendiquées pour leur activité diurétique et sodiurétique.

Les composés objets de l'invention sont représentés par la formule générale **I** 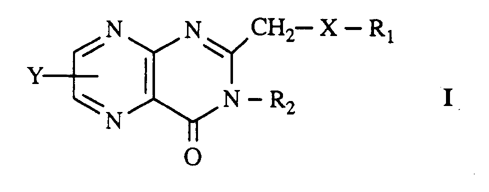
dans laquelle X est un atome d'oxygène ou un atome de soufre, Y est un atome d'hydrogène, un radical alcoyle inférieur, en particulier un radical méthyle, en position 6 ou un groupe hydroxyle en position 7, R₁ est un atome d'hydrogène, un radical alcoyle inférieur, un radical phényle substitué ou non, un radical benzyle, le groupe méthoxyméthyle, le groupe acétyle, le groupe 2-acétoxyéthyle ou le groupe 2,2,2-trifluoroéthyle et R₂ est un atome d'hydrogène ou un radical alcoyle inférieur en particulier un radical méthyle.

Le terme inférieur appliqué à un radical alcoyle signifie que le radical peut être linéaire ou ramifié et qu'il peut comprendre de 1 à 4 atomes de carbone.

Le terme substitué appliqué à un radical phényle signifie que le radical peut être substitué par un à trois groupes choisis parmi alcoyle inférieur, alcoxy inférieur, halogène, hydroxyle et acétyle.

Les formes tautomères éventuelles des composés de l'invention, font partie intégrante de l'invention. A titre d'exemple, mais non exclusivement, lorsque Y est un groupe hydroxyle en position 7, les composés de l'invention de formule **Ia** peuvent s'écrire aussi selon la forme tautomère de formule **Ib**.

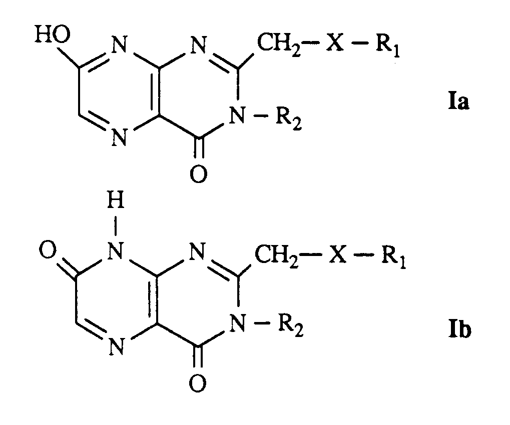

Les composés dans la formule desquels X est un atome d'oxygène, Y est un atome d'hydrogène ou un groupe hydroxyle en position 7 et R₂ est un atome d'hydrogène constituent une classe particulièrement intéressante.

Les radicaux R₁ préférés sont les radicaux alcoyles inférieurs, le radical phényle ou le radical benzyle.

Les sels pharmaceutiquement acceptables font également partie intégrante de l'invention. Ce peuvent être des sels de métaux alcalins. Ces sels sont obtenus en traitant les composés de l'invention par des hydroxydes ou des carbonates de métaux alcalins. Par métaux alcalins, on entend des métaux comme le sodium ou le potassium.

Les composés de l'invention peuvent se préparer selon l'une au moins des méthodes suivantes :
a) Un 3-aminopyrazine-2-carboxamide de formule **II** 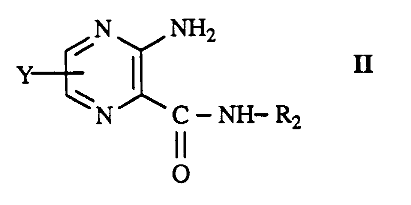 peut être condensé sur un orthoester de formule **III**

   R₁XCH₂C(OR₃)₃ **III**

   Dans les formules **II** et **III**, X, Y, R₁ et R₂ ont les significations données précédemment. Dans la formule **III**, R₃ est un radical alcoyle inférieur, de préférence le radical éthyle. La réaction s'effectue dans l'anhydride acétique en présence d'un excès d'orthoester de formule **III**. La température est la température d'ébullition du milieu réactionnel. Le temps de réaction est généralement compris entre 1 et 3 heures.
b) Lorsque R₂ est un atome d'hydrogène, une 4-aminoptéridine de formule **IV** 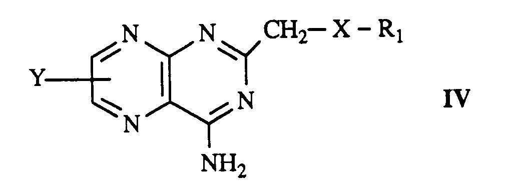 peut être hydrolysée dans l'eau alcaline. Dans la formule **IV**, X, Y et R₁ ont les significations données précédemment. La réaction s'effectue dans l'eau en présence d'une base telle que la soude ou la potasse. La température peut varier entre la température ambiante et la température d'ébullition du milieu réactionnel, préférentiellement entre 75°C et la température d'ébullition du milieu réactionnel. Le temps de réaction est généralement compris entre 10 minutes et 14 heures.
c) Lorsque Y et R₂ sont des atomes d'hydrogène, une 5,6-diaminopyrimidin-4(3H)-one de formule **V** 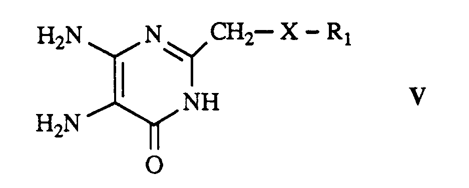 est condensée avec le glyoxal. Dans la formule **V**, X et R₁ ont les significations données précédemment. La réaction s'effectue dans l'eau. La température peut varier entre la température ambiante et la température d'ébullition du milieu réactionnel. Le temps de réaction est généralement compris entre 1 et 24 heures.
d) Dans le cas particulier où X est un atome d'oxygène, Y un atome d'hydrogène, R₁ le groupe 2-acétoxyéthyle et R₂ un atome d'hydrogène, le composé de l'invention **I** correspondant peut être obtenu par l'hydrolyse suivie de l'acétylation du composé **IV** pour lequel X est un atome d'oxygène, Y un atome d'hydrogène et R₁ le groupe 2-hydroxyéthyle.
e) Dans le cas particulier où X est un atome d'oxygène, Y un groupe hydroxyle en position 7 et où R₁ et R₂ sont des atomes d'hydrogène, le composé de l'invention **I** correspondant peut être obtenu par hydrolyse du composé de même formule excepté R₁ qui représente le groupe acétyle.

Les 3-aminopyrazine-2-carboxamides intermédiaires de formule **II** sont des composés connus. Les orthoesters intermédiaires de formule **III** sont pour la plupart d'entre eux des composés connus. Ceux qui sont nouveaux ont été préparés selon les techniques usuelles décrites notamment par S.M. Mc Elvain et J.W. Nelson, J. Am. Chem. Soc. 1942, 64, 1825.

Les 4-aminoptéridines intermédiaires de formule **IV** sont des composés nouveaux. Elles sont préparées par condensation d'un 3-aminopyrazine-2-carbonitrile de formule **VI**. 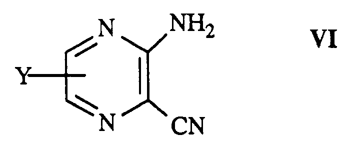
sur une acétamidine de formule **VII** 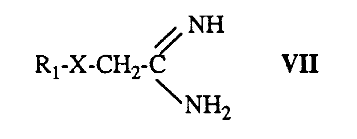
Dans les formules **VI** et **VII**, X, Y et R₁ ont les significations données précédemment.

Dans le cas particulier où X est un atome d'oxygène, Y un groupe hydroxyle en position 7 et R₁ le groupe méthoxyméthyle, la 4-aminoptéridine correspondante **XI** est obtenue selon le schéma réactionnel ci-dessous : 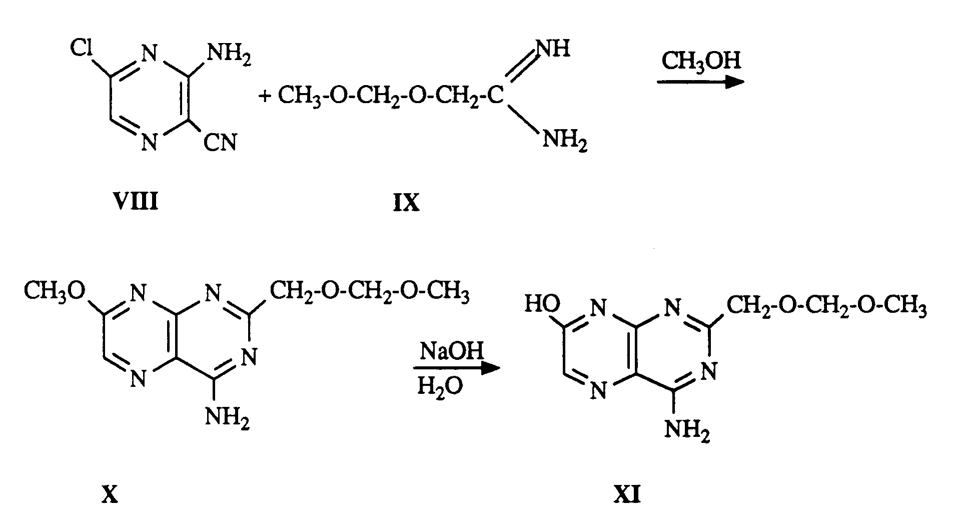
Le 3-amino-5-chloropyrazine-2-carbonitrile **VIII** est condensé sur la 2-(méthoxyméthoxy)acétamidine **IX** en présence de méthanol pour donner la 4-amino-7-méthoxy-2-(méthoxyméthoxyméthyl)ptéridine de formule **X**. Le groupe méthoxy en position 7 du composé **X** peut être hydrolysé sélectivement en milieu alcalin pour fournir la 4-amino-2-(méthoxyméthoxyméthyl)ptéridin-7-ol de formule **XI**.

Les 3-aminopyrazine-2-carbonitriles intermédiaires de formule **VI** sont des composés connus. Les acétamidines intermédiaires de formule **VII** sont pour certaines d'entre elles des composés connus. Celles qui sont nouvelles ont été préparées selon les techniques usuelles par réaction de l'ammoniac sur les acétimidates d'alcoyle de formule **XII** 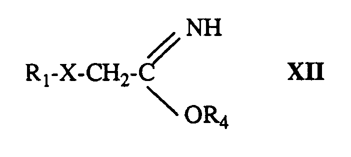
dans un alcool de bas poids moléculaire anhydre, comme le méthanol ou l'éthanol. Dans la formule **XII**, X et R₁ ont les significations données précédemment et R₄ est un radical alcoyle, de préférence le radical méthyle ou le radical éthyle.

Les acétimidates d'alcoyle intermédiaires de formule **XII** sont pour certains d'entre eux des composés connus. Ceux qui sont nouveaux ont été préparés selon les techniques usuelles décrites notamment par C. Djerassi et C.R. Scholz, J. Am. Chem. Soc. 1947, 69, 1688 et par F.C. Schaefer et G.A. Peters, J. Org. Chem. 1961, 26, 412.

Dans le cas particulier où X est un atome d'oxygène, R₁ le groupe 2-hydroxyéthyle et R₄ le radical éthyle, l'acétimidate d'alcoyle **XII** correspondant est obtenu selon une variante de la méthode de F.C. Schaefer et G.A. Peters, à partir du (2-acétoxyéthoxy)acétonitrile.

Dans le cas particulier où X est un atome d'oxygène et ou R₁ et R₄ sont des radicaux éthyles, l'acétimidate d'alcoyle **XII** correspondant peut être obtenu par réaction du chloroacétonitrile sur l'éthylate de sodium et l'éthanol.

Les 5,6-diaminopyrimidin-4(3H)-ones de formule **V** sont des composés nouveaux. Elles sont préparées selon le schéma réactionnel ci-dessous : 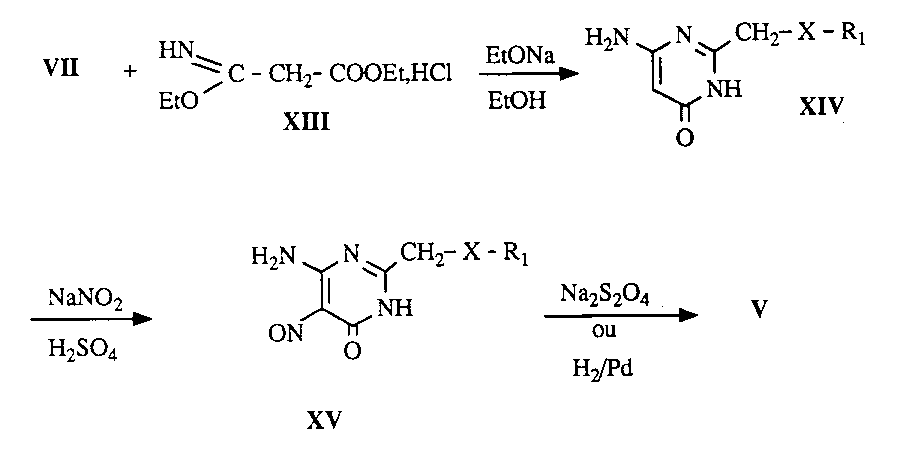
Une acétamidine de formule **VII** est condensée sur l'iminoéther du cyanoacétate d'éthyle **XIII** pour donner une 6-aminopyrimidin-4(3H)-one de formule **XIV**. Ces composés **XIV** sont nitrosés en 6-amino-5-nitrosopyrimidin-4(3H)-ones de formule **XV**. La réduction des composés de formule **XV** par le dithionite de sodium ou par hydrogénation catalytique fournit les 5,6-diaminopyrimidin-4(3H)-ones **V.**

Les composés représentés par la formule générale **I** possèdent d'excellentes propriétés antiallergiques et sont supérieurs aux produits connus, en particulier par le fait qu'ils sont actifs en administration orale.

L'activité antiallergique a été mesurée chez le rat par le test d'anaphylaxie cutanée passive ou PCA décrit par I. Mota, Life Sciences 1963, 7, 465 et Z. Ovary et al., Proceedings of Society of Experimental Biology and Medecine 1952, 81, 584.

Dans ce test, la peau des rats est sensibilisée par 4 injections intradermiques d'un anti-sérum homologue d'ovalbumine dilué au 1/15^{e}. Le jour suivant la sensibilisation, les animaux reçoivent simultanément en injection intraveineuse, 1 ml/kg d'une solution saline à 2,5 % de bleu Evans et 1 ml/kg d'une solution saline à 2,5 % d'ovalbumine. Les composés testés sont administrés avant l'antigène, le délai étant de 5 mn en IP et 10 mn PO. Trente minutes après injection du mélange ovalbumine/colorant, les animaux sont sacrifiés et l'intensité de la réponse allergique est déterminée en mesurant l'étendue de la coloration cutanée. La protection apportée par les produits de l'invention est exprimée en DE₅₀ (dose diminuant la surface cutanée du colorant de 50 %). Quatre rats sont utilisés pour chaque dose de produit.

Les résultats obtenus par voie intrapéritonéale pour quelques produits de l'invention sont consignés dans le tableau I.

**Tableau I**

| PRODUITS | PCA DE₅₀(mg/kg/IP) |
|---|---|
| Exemple 1 | 7 |
| Exemple 3 | 14 |
| Exemple 4 | 9 |
| Exemple 7 | 21 |
| Exemple 9 | 39 |
| Exemple 11 | 10 |
| Exemple 12 | 18 |
| Exemple 13 | 14 |

Les produits présentant les meilleurs résultats par voie intrapéritonéale ont été testés par voie orale. Ces résultats sont consignés dans le tableau II.

**Tableau II**

| PRODUITS | PCA DE₅₀ (mg/kg/PO) |
|---|---|
| Exemple 1 | 24 |
| Exemple 3 | 33 |
| Exemple 4 | 25 |
| Exemple 11 | 48 |

Les composés décrits se distinguent en outre des produits connus par leur longue durée d'action et par la faculté d'antagoniser les effets du PAF-acéther, spécialement ses effets bronchoconstricteurs. A titre d'illustration, la DE₅₀ par voie intraveineuse du composé décrit dans l'exemple 1 est de 0,068 mg/kg sur le bronchospasme induit, chez le cobaye anesthésié, par injection de 10 ng/kg/IV de PAF-acéther.

Les composés de l'invention manifestent une faible toxicité. A titre d'illustration, pour le composé décrit dans l'exemple 1, les doses létales 50 déterminées sur le rat et sur la souris par voie orale sont supérieures à 2000 mg/kg et les doses létales 50 déterminées sur la souris par voie intrapéritonéale et par voie intraveineuse sont supérieures à 1600 mg/kg.

La présente demande a également pour objet l'application des composés **I** à titre de médicaments et notamment de médicaments antiallergiques. Ces médicaments peuvent être administrés par inhalation sous forme d'aérosols, par voie orale sous forme de comprimés, comprimés dragéifiés ou de gélules, par voie intraveineuse sous forme de soluté injectable, par voie cutanée sous forme de pommade, de poudre ou de solution, ou par voie rectale sous forme de suppositoires. Les posologies journalières peuvent varier de 1 à 50 mg de principe actif pris par inhalation, de 5 à 250 mg de principe actif pris par voie orale, de 1 à 50 mg de principe actif pris par voie intraveineuse et de 20 à 400 mg de principe actif pris par voie rectale.

On donne ci-dessous à titre d'exemples non limitatifs quelques formulations pharmaceutiques :

| - Composition d'une capsule pour inhalation : | |
|---|---|
| principe actif | 5 mg |

| - Composition d'un aérosol : | |
|---|---|
| principe actif | 1 g |
| gaz propulseurs | 99 g |

| - Composition d'un comprimé : | |
|---|---|
| principe actif | 50 mg |
| excipient : lactose, amidon de blé, polyvidone, talc, stéarate de magnésium | |

| - Composition d'une gélule : | |
|---|---|
| principe actif | 50 mg |
| excipient : lactose, amidon de blé, talc, stéarate de magnésium | |

| - Composition d'une ampoule de soluté injectable : | |
|---|---|
| principe actif | 10 mg |
| excipient : sorbitol, eau pour préparations injectables qsp | 5 ml |

| - Composition d'un suppositoire : | |
|---|---|
| principe actif | 50 mg |
| glycérides semi-synthétiques qsp | 500 mg |

| - Composition d'une pommade : | |
|---|---|
| principe actif | 0,5 % |
| stéarate de glycérol et de polyoxyéthylèneglycol | 15 % |
| glycérides saturés polyoxyéthylénés | 2 % |
| huile de vaseline | 6 % |
| eau qsp | 100% |

Les exemples suivants illustrent l'invention. Dans les données de résonance magnétique nucléaire (R.M.N.), les abréviations suivantes ont été utilisées : s pour singulet, d pour doublet, t pour triplet, q pour quadruplet et m pour massif complexe ; les déplacements chimiques δ sont exprimés en ppm.

### Exemple 1 :

### 2-(Ethoxyméthyl)ptéridin-4(3H)-one

Un mélange de 8,3 g (0,060 mole) de 3-aminopyrazine-2-carboxamide (préparé selon R.C. Ellingson et ai., J. Am. Chem. Soc. 1945, 67, 1711), de 49,5 g (0,24 mole) d'orthoéthoxyacétate de triéthyle (préparé selon S.M. Mc Elvain et P.M. Walters, J. Am. Chem. Soc. 1942, 64, 1963) et de 53 ml d'anhydride acétique est porté à reflux sous atmosphère d'azote pendant 3 heures. La température de ce reflux baisse au cours du temps de 124 à 96°C, puis reste constante à cette dernière température. Après refroidissement, le précipité formé est isolé par filtration. Il est lavé à l'éther et recristallisé dans l'éthanol. Rdt : 5,5 g (44 %), F = 168 - 169°C.

| Analyse centésimale : C₉H₁₀N₄O₂ (M = 206,21) | | | |
|---|---|---|---|
| | C % | H % | N % |
| calculé | 52,42 | 4,89 | 27,17 |
| trouvé | 52,14 | 4,91 | 27,34 |

IR : ν̅ (C=O) = 1690 cm⁻¹
R.M.N. (CDCl₃) : δ = 1,3 (3H,t) ; 3,7 (2H,q) ; 4,6 (2H,s) ; 8,8 (1H,d) ; 8,9 (1H,d) ; 10,3 (1H,pic échangeable avec CF₃COOD).

### Exemple 2 :

### 2-(Ethoxyméthyl)ptéridin-4(3H)-one

### a) 2-Ethoxyacétimidate de méthyle

On ajoute 66,7 g (0,78 mole) d'éthoxyacétonitrile (préparé selon L. Ramachandra Row et T.R. Thiruvengadam, Current Sci. (India) 1947, 16, 379) à une solution de méthylate de sodium dans le méthanol, obtenue par réaction de 1,8 g (0,078 atome-gramme) de sodium dans 400 ml de méthanol. Le milieu réactionnel est agité pendant 3 jours à température ambiante. Le méthylate de sodium est alors neutralisé par un courant de gaz carbonique, puis le milieu réactionnel est concentré sous pression réduite. Le résidu obtenu est repris à l'éther. Les produits minéraux sont éliminés par filtration ; le filtrat éthéré est concentré et le résidu est distillé sous pression réduite. Rdt : 53,5 g (59 %), E₁₅ = 38-40°C.
R.M.N. (CDCl₃) : δ = 1,2 (3H,t) ; 3,5 (2H,q) ; 3,7 (3H,s) ; 3,8 (2H,s) ; 7,6 (1H,pic échangeable avec D₂O).

### b) 2-Ethoxyacétamidine

A une solution, maintenue à 10°C, de 39 g (2,29 moles) d'ammoniac dans 1225 ml d'éthanol absolu, on ajoute 53,5 g (0,457 mole) de 2-éthoxyacétimidate de méthyle. La solution obtenue est abandonnée pendant 6 jours à température ambiante, puis est concentrée sous pression réduite. Elle est utilisée dans l'étape suivante sans autre purification.
Rdt : 46,7 g (quantitatif).

### c) 4-Amino-2-(éthoxyméthyl)ptéridine

Un mélange de 36,0 g (0,30 mole) de 3-aminopyrazine-2-carbonitrile (préparé selon A. Albert et K. Ohta, J. Chem. Soc. C, 1970, 1540) et de 46,7 g de 2-éthoxyacétamidine dans 700 ml d'éthanol absolu est porté à reflux pendant 2 heures 30 sous atmosphère d'azote. Après refroidissement, le précipité obtenu est isolé par filtration. Il est purifié par recristallisation dans l'éthanol. Rdt : 52,0 g (84 %), F = 152 - 154°C.

| Analyse centésimale : C₉H₁₁N₅O (M = 205,22) | | | |
|---|---|---|---|
| | C % | H % | N % |
| calculé | 52,67 | 5,40 | 34,13 |
| trouvé | 52,56 | 5,37 | 34,04 |

R.M.N. (DMSO d₆) : δ = 1,1 (3H,t) ; 3,5 (2H,q) ; 4,4 (2H,s) ; 8,2 (2H,pic échangeable avec CF₃COOD) ; 8,7 (1H,d) ; 9,0 (1H,d).

### d) 2-(Ethoxyméthyl)ptéridin-4(3H)-one

Une solution de 30,0 g (0,146 mole) de 4-amino-2-(éthoxyméthyl)ptéridine dans 800 ml de soude 5 % est portée lentement à 75°C et maintenue à cette température pendant 2 heures. Après refroidissement, la solution obtenue est acidifiée par l'acide acétique jusqu'à un pH égal à 6, puis est extraite au chloroforme. Les extraits organiques sont séchés sur sulfate de sodium et concentrés à sec sous pression réduite. Le résidu solide est recristallisé dans l'éthanol en présence de Norit. Rdt : 19,5 g (65 %), F = 168 - 169°C. Le produit est identique à celui obtenu dans l'exemple 1.

### Exemple 3 :

### 2-(Méthoxyméthyl)ptéridin-4(3H)-one

Obtenue en opérant comme dans l'exemple 1 à partir de 11,7 g (0,085 mole) de 3-aminopyrazine-2-carboxamide, de 73,0 g (0,38 mole) d'orthométhoxyacétate de méthyle (préparé selon E.T. Stiller, brevet U.S. 2.422.598 ; C.A., 1947, 41, 5904a) et de 75 ml d'anhydride acétique. Rdt : 12,3 g (75 %), F = 187 - 189°C (éthanol).

| Analyse centésimale : C₈H₈N₄O₂ (M = 192,18) | | | |
|---|---|---|---|
| | C % | H % | N % |
| calculé | 50,00 | 4,20 | 29,15 |
| trouvé | 49,92 | 4,10 | 29,12 |

IR : ν̅ (C = O) = 1690 cm⁻¹
R.M.N. (DMSO d₆) : δ = 3,4 (3H,s) ; 4,4 (2H,s) ; 8,8 (1H,d) ; 9,0 (1H,d) ; 12,7 (1H,pic échangeable avec CF₃COOD).

### Exemple 4 :

### 2-(Propoxyméthyl)ptéridin-4(3H)-one

### a) Chlorhydrate de 2-propoxyacétimidate d'éthyle

Dans une solution, refroidie à 0°C, de 29,7 g (0,30 mole) de propoxyacétonitrile (préparé selon D. Gauthier, Compt Rend. Acad. Sci. 1906, 143, 831) et de 13,8 g (0,30 mole) d'éthanol absolu dans 500 ml d'éther, on fait barboter, pendant une heure, du gaz chlorhydrique. Le milieu réactionnel est ensuite abandonné pendant 2 jours à 0°C, puis est repris par 300 ml d'éther. Le précipité formé est isolé par filtration. Il est lavé à l'éther et séché sous pression réduite ; il est utilisé dans l'étape suivante sans autre purification. Rdt : 30,2 g (55 %).

### b) Orthopropoxyacétate de triéthyle

On dissout 30,2 g (0,17 mole) de chlorhydrate de 2-propoxyacétimidate d'éthyle dans 160 ml d'éthanol absolu. La solution est abandonnée pendant 2 jours à température ambiante ; elle est ensuite concentrée sous pression réduite. Le résidu obtenu est repris à l'éther et filtré. La solution éthérée est séchée sur carbonate de potassium, filtrée et concentrée sous pression réduite. Le liquide obtenu est utilisé dans l'étape suivante sans autre purification. Rdt : 29,3 g (80 %).
R.M.N. (CDCl₃) : δ = 0,9 (3H,t) ; 1,3 (9H,t) ; 1,3 - 2,0 (2H,m) ; 3,4 (2H,t) ; 3,5 (2H,s) ; 3,6 (6H,q).

### c) 2-(Propoxyméthyl)ptéridin-4(3H)-one

Obtenue en opérant comme dans l'exemple 1 à partir de 4,6 g (0,033 mole) de 3-aminopyrazine-2-carboxamide, de 29,3 g (0,133 mole) d'orthopropoxyacétate de triéthyle et de 30 ml d'anhydride acétique. Rdt : 2,6 g (36 %), F = 158 - 160°C (éthanol).

| Analyse centésimale : C₁₀H₁₂N₄O₂ (M = 220,23) | | | |
|---|---|---|---|
| | C % | H % | N % |
| calculé | 54,54 | 5,49 | 25,44 |
| trouvé | 54,55 | 5,45 | 25,68 |

IR : ν̅ (C = O) = 1690 cm⁻¹
R.M.N. (DMSO d₆) : δ = 0,9 (3H,t) ; 1,3 - 2,0 (2H,m) ; 3,5 (2H,t) ; 4,4 (2H,s) ; 8,8 (1H,d) ; 9,0 (1H,d) ; 12,7 (1H,pic échangeable avec CF₃COOD).

### Exemple 5 :

### 2-(Méthoxyméthyl)-3-méthylptéridin-4(3H)-one

Un mélange de 7,8 g (0,0513 mole) de 3-amino-N-méthylpyrazine-2-carboxamide (préparé selon W.F. Keir et al., J. Chem. Soc., Perkin Trans 1, 1978, 1002), de 39,4 g (0,205 mole) d'orthométhoxyacétate de triéthyle et de 45,3 ml d'anhydride acétique est porté à reflux sous atmosphère d'azote pendant 3 heures. La température de ce reflux baisse au cours du temps de 114 à 90°C, puis reste constante à cette dernière température. Après refroidissement, le milieu réactionnel est concentré à sec sous pression réduite. Le résidu obtenu est purifié par chromatographie sur colonne de silice (éluant : chloroforme-méthanol, 95-5 puis 90-10), puis est recristallisé dans l'acétate d'éthyle. Rdt : 5,0 g (47 %), F = 130,5 - 132,5°C.

| Analyse centésimale : C₉H₁₀N₄O₂ (M = 206,21) | | | |
|---|---|---|---|
| | C % | H % | N % |
| calculé | 52,42 | 4,89 | 27,17 |
| trouvé | 52,51 | 4,94 | 27,17 |

IR : ν̅ (C = O) = 1690 cm⁻¹
R.M.N. (DMSO d₆) : δ = 3,4 (3H,s) ; 3,6 (3H,s) ; 4,7 (2H,s) ; 8,9 (1H,d) ; 9,0 (1H,d).

### Exemple 6 :

### 2-(Acétoxyméthyl)ptéridin-4(3H)-one

### a) Chlorhydrate de 2-acétoxyacétimidate d'éthyle

Dans une solution, refroidie à 0°C, de 30,0 g (0,303 mole) d'acétoxyacétonitrile (préparé selon L. Henry, Rec. Trav. Chim. Pays Bas 1905, 24, 169) et de 15,4 g (0,334 mole) d'éthanol absolu dans 300 ml d'éther, on fait barboter du gaz chlorhydrique jusqu'à saturation. Le milieu réactionnel est ensuite abandonné pendant 5 heures à 0°C. Le précipité formé est isolé par filtration. Il est lavé à l'éther et séché sous pression réduite ; il est utilisé dans l'étape suivante sans autre purification. Rdt : 52,2 g (95 %), F = 100 - 101°C.
R.M.N. (DMSO d₆ + CF₃COOD) : δ = 1,1 (3H,t) ; 1,7 (3H,s) ; 3,4 (2H,q) ; 3,8 (2H,s).

### b) Orthoacétoxyacétate de triéthyle

Un mélange de 34,0 g (0,187 mole) de chlorhydrate de 2-acétoxyacétimidate d'éthyle et de 340 ml d'éthanol absolu est abandonné pendant 3 jours à température ambiante. Le précipité formé est éliminé par filtration ; le filtrat est concentré sous pression réduite. Le résidu obtenu est repris par 800 ml d'éther et refroidi vers - 10°C. Il se forme un nouveau précipité qui est éliminé par filtration. Le filtrat est concentré sous pression réduite. Le liquide obtenu est utilisé dans l'étape suivante sans autre purification. Rdt : 34,5 g (84 %).
R.M.N. (CDCl₃) : δ = 1,0 (9H,t) ; 1,9 (3H,s) ; 3,4 (6H,q) ; 4,1 (2H,s).

### c) 2-(Acétoxyméthyl)ptéridin-4(3H)-one

Obtenue en opérant comme dans l'exemple 1 à partir de 5,3 g (0,038 mole) de 3-aminopyrazine-2-carboxamide, de 34,5 g (0,157 mole) d'orthoacétoxyacétate de triéthyle et de 34,5 ml d'anhydride acétique. Durée du reflux : 1 heure 45. Rdt : 5,4 g (65 %), F = 210 - 212°C (éthanol).

| Analyse centésimale : C₉H₈N₄O₃ (M = 220,19) | | | |
|---|---|---|---|
| | C % | H % | N % |
| calculé | 49,09 | 3,66 | 25,45 |
| trouvé | 49,02 | 3,73 | 25,56 |

IR : ν̅ (C = O) = 1670 et 1720 cm⁻¹
R.M.N. (DMSO d₆) : δ = 2,2 (3H,s) ; 5,0 (2H,s) ; 8,8 (1H,d) ; 9,0 (1H,d) ; 13,8 (pic échangeable avec CF₃COOD).

### Exemple 7 :

### 2-(Ethoxyméthyl)-7-hydroxyptéridin-4(3H)-one

Obtenue en opérant comme dans l'exemple 1 à partir de 2,1 g (0,0136 mole) de 3-amino-5-hydroxypyrazine-2-carboxamide (préparé selon E.C. Taylor et al., J. Org. Chem. 1975, 40, 2341 ), de 14,0 g (0,068 mole) d'orthoéthoxyacétate de triéthyle et de 14,0 ml d'anhydride acétique. Rdt : 1,2 g (40 %), F = 255 - 256°C (méthanol - N,N-diméthylformamide).

| Analyse centésimale : C₉H₁₀N₄O₃ (M = 222,20) | | | |
|---|---|---|---|
| | C % | H % | N % |
| calculé | 48,65 | 4,54 | 25,21 |
| trouvé | 48,88 | 4,56 | 25,14 |

IR : ν̅ (C = O) = 1640 et 1690 cm⁻¹
R.M.N. (DMSO d₆) : δ = 1,1 (3H,t) ; 3,5 (2H,q) ; 4,3 (2H,s) ; 7,9 (1H,s) ; 12,7 (2H,pic élargi).

### Exemple 8 :

### 2-(Acétoxyméthyl)-7-hydroxyptéridin-4(3H)-one

Obtenue en opérant comme dans l'exemple 1 à partir de 5,0 g (0,0324 mole) de 3-amino-5-hydroxypyrazine-2-carboxamide, de 29,5 g (0,134 mole) d'orthoacétoxyacétate de triéthyle et de 9,5 ml d'anhydride acétique. Durée du reflux : 1 heure 45. Rdt : 3,8 g (50 %), F > 300°C (éthanol- N,N-diméthylformamide).

| Analyse centésimale : C₉H₈N₄O₄ (M = 236,19) | | | |
|---|---|---|---|
| | C % | H % | N % |
| calculé | 45,77 | 3,41 | 23,72 |
| trouvé | 45,79 | 3,65 | 23,84 |

IR : ν̅ (C = O) = 1625, 1690 et 1735 cm⁻¹
R.M.N. (DMSO d₆) : δ = 2,2 (3H,s) ; 5,0 (2H,s) ; 7,9 (1H,s) ; 12,9 (2H,pic échangeable avec CF₃COOD).

### Exemple 9 :

### 2-(Phénoxyméthyl)ptéridin-4(3H)-one

### a) 4-Amino-2-(phénoxyméthyl)ptéridine

Obtenue en opérant comme dans le paragraphe c de l'exemple 2, à partir de 3,0 g (0,025 mole) de 3-aminopyrazine-2-carbonitrile et de 3,8 g (0,025 mole) de 2-phénoxyacétamidine (préparée selon C. Djerassi et C.R. Scholz, J. Am. Chem. Soc. 1947, 69, 1688 ) dans 100 ml d'éthanol absolu. Durée du reflux : 4 heures. Rdt : 5,4 g (85 %), F = 202 - 204°C (éthanol).

| Analyse centésimale : C₁₃H₁₁N₅O (M = 253,26) | | | |
|---|---|---|---|
| | C % | H % | N % |
| calculé | 61,65 | 4,38 | 27,65 |
| trouvé | 61,28 | 4,22 | 27,37 |

R.M.N. (DMSO d₆) : δ = 5,1 (2H,s) ; 6,6 - 7,6 (5H,m) ; 8,4 (2H,pic échangeable avec CF₃OOOD) ; 8,8 (1H,d) ; 9,0 (1H,d).

### b) 2-(Phénoxyméthyl)ptéridin-4(3H)-one

Un mélange de 4,0 g (0,016 mole) de 4-amino-2-(phénoxyméthyl)ptéridine et de 400 ml de soude 5 % est porté lentement à 95°C et maintenu à cette température pendant 2 heures. Après refroidissement, la solution obtenue est acidifiée par l'acide acétique jusqu'à un pH égal à 5,5. Le précipité formé est isolé par filtration. Il est purifié par recristallisation dans l'éthanol. Rdt : 2,8 g (70 %), F = 220 - 221°C.

| Analyse centésimale : C₁₃H₁₀N₄O₂ (M = 254,25) | | | |
|---|---|---|---|
| | C % | H % | N % |
| calculé | 61,41 | 3,96 | 22,04 |
| trouvé | 61,44 | 3,97 | 22,08 |

IR : ν̅ (C = O) = 1690 cm⁻¹
R.M.N. (DMSO d₆) : δ = 5,0 (2H,s) ; 6,7 - 7,4 (5H,m) ; 8,7 (1H,d) ; 8,9 (1H,d) ; 12,8 (1H,pic échangeable avec CF₃COOD).

### Exemple 10 :

### 2-[(2,3-Dichlorophénoxy)méthyl]ptéridin-4(3H)-one

### a) Chlorhydrate de 2-(2,3-dichlorophénoxy)acétimidate d'éthyle

Obtenu en opérant comme dans le paragraphe a de l'exemple 6, à partir de 219,8 g (1,086 mole) de (2,3-dichlorophénoxy) acétonitrile (préparé selon R.W. Fuller et al., J. Med. Chem. 1973, 16, 101) et de 50,0 g (1,086 mole) d'éthanol absolu dans 1750 ml d'éther. Durée de la réaction : 16 heures à 0°C. Rdt : 293,5 g (95 %), F = 167 - 169°C.
R.M.N. (DMSO d₆) : δ = 1,3 (3H,t) ; 4,3 (2H,q) ; 5,1 (2H,s) ; 6,6 (1H,s échangeable avec CF₃COOD) ; 7,0 - 7,6 (3H,m) ; 8,3 (1H,s échangeable avec CF₃COOD).

### b) Chlorhydrate de 2-(2,3-dichlorophénoxy)acétamidine

A une solution, refroidie à 10°C, de 3,5 g (0,21 mole) d'ammoniac dans 100 ml d'éthanol absolu, on ajoute rapidement 11,8 g (0,041 mole) de chlorhydrate de 2-(2,3-dichlorophénoxy)acétimidate d'éthyle. Le milieu réactionnel est abandonné pendant 3 jours à température ambiante. Après élimination par filtration de quelques particules en suspension, le milieu réactionnel est concentré à sec sous pression réduite. Le résidu solide obtenu est purifié par lavage à l'éther et recristallisation dans l'isopropanol. Rdt : 8,0 g (75 %), F = 204,5 - 206,5°C.

| Analyse centésimale : C₈H₉Cl₃N₂O (M = 255,53) | | | | |
|---|---|---|---|---|
| | C % | H % | Cl % | N % |
| calculé | 37,60 | 3,55 | 41,62 | 10,96 |
| trouvé | 37,61 | 3,60 | 41,36 | 10,85 |

R.M.N. (DMSO d₆ + D₂O) : δ = 5,1 (2H,s) ; 7,1 - 7,7 (3H,m)

### c) 2-(2,3-Dichlorophénoxy)acétamidine

On met en suspension 8,0 g (0,031 mole) de chlorhydrate de 2-(2,3-dichlomphénoxy)acétamidine dans une solution de soude et extrait au chloroforme. L'extrait organique est concentré à sec sous pression réduite. Le résidu solide est lavé à l'hexane et séché ; il est utilisé dans l'étape suivante sans autre purification. Rdt : 6,7 g (98 %), F = 104 - 108°C.

### d) 4-Amino-2-[(2,3-dichlorophénoxy)méthyl]ptéridine

Obtenue en opérant comme dans le paragraphe c de l'exemple 2, à partir de 3,6 g (0,030 mole) de 3-aminopyrazine-2-carbonitrile et de 6,6 g (0,030 mole) de 2-(2,3-dichlorophénoxy)acétamidine dans 100 ml d'éthanol absolu. Durée du reflux : 14 heures. Rdt : 8,1 g (84%), F = 216 - 218°C (éthanol).

| Analyse centésimale : C₁₃H₉Cl₂N₅O (M = 322,15) | | | | |
|---|---|---|---|---|
| | C % | H % | Cl % | N % |
| calculé | 48,47 | 2,82 | 22,01 | 21,74 |
| trouvé | 48,38 | 2,86 | 22,10 | 21,81 |

R.M.N. (DMSO d₆) : δ = 5,2 (2H,s) ; 7,1 (3H,s) ; 8,3 (2H, pic échangeable avec CF₃COOD) ; 8,7 (1H,d) ; 9,0 (1H,d).

### e) 2-[(2,3-Dichlorophénoxy)méthyl]ptéridin-4(3H)-one

Obtenue en opérant comme dans le paragraphe b de l'exemple 9, à partir de 1,0 g (0,0031 mole) de 4-amino-2-[(2,3-dichlorophénoxy)méthyl]ptéridine dans 35 ml de soude 5 %. Durée du chauffage : 10 minutes à reflux. Rdt : 0,5 g (50 %), F = 219 - 220,5°C (éthanol).

| Analyse centésimale : C₁₃H₈Cl₂N₄O₂ (M = 323,14) | | | | |
|---|---|---|---|---|
| | C % | H % | Cl % | N % |
| calculé | 48,32 | 2,50 | 21,94 | 17,34 |
| trouvé | 48,53 | 2,53 | 21,99 | 17,49 |

IR : ν̅ (C = O) = 1680 cm⁻¹
R.M.N. (DMSO d₆ + CF₃COOD) : δ = 5,1 (2H,s) ; 7,1 (3H,s) ; 8,7 (1H,d) ; 8,9 (1H,d).

### Exemple 11 :

### 2-(Benzyloxyméthyl)ptéridin-4(3H)-one

### a) 4-Amino-2-(benzyloxyméthyl)ptéridine

Obtenue en opérant comme dans le paragraphe c de l'exemple 2, à partir de 12,0 g (0,10 mole) de 3-aminopyrazine-2-carbonitrile et de 25,0 g (0,15 mole) de 2-(benzyloxy)acétamidine (préparée selon W.J. Haggerty Jr. et W.J. Rost, J. Pharm. Sci. 1969, 58, 50) dans 400 ml d'éthanol absolu. Durée du reflux : 4 heures. Rdt : 15,4 g (58 %), F = 112 - 114°C.Un échantillon analytique a été obtenu par recristallisation dans l'éthanol, puis lavage à l'acide chlorhydrique dilué et enfin recristallisation dans l'acétate d'éthyle. F = 131 - 133°C.

| Analyse centésimale : C₁₄H₁₃N₅O (M = 267,29) | | | |
|---|---|---|---|
| | C % | H % | N % |
| calculé | 62,91 | 4,90 | 26,20 |
| trouvé | 62,74 | 4,79 | 26,22 |

R.M.N. (DMSO d₆) : δ = 4,6 (2H,s) ; 4,7 (2H,s) ; 7,1 - 7,6 (5H,m) ; 8,3 (2H,pic échangeable avec CF₃COOD) ; 8,8 (1H,d) ; 9,0 (1H,d).

### b) 2-(Benzyloxyméthyl)ptéridin-4(3H)-one

Obtenue en opérant comme dans le paragraphe b de l'exemple 9, à partir de 10,0 g (0,037 mole) de 4-amino-2-(benzyloxyméthyl)ptéridine brute dans 300 ml de soude 5 %. Durée du chauffage : 2 heures à 80°C. Rdt : 8,2 g (83 %), F = 157 - 159°C (éthanol).

| Analyse centésimale : C₁₄H₁₂N₄O₂ (M = 268,28) | | | |
|---|---|---|---|
| | C % | H % | N % |
| calculé | 62,68 | 4,51 | 20,88 |
| trouvé | 62,85 | 4,39 | 21,15 |

IR : ν̅ (C = O) = 1680 cm⁻¹
R.M.N. (DMSO d₆) : δ = 4,5 (2H,s) ; 4,6 (2H,s) ; 7,1 - 7,6 (5H,m) ; 8,8 (1H,d) ; 8,9 (1H,d) ; 12,7 (1H,pic échangeable avec CF₃COOD).

### Exemple 12 :

### 2-(Méthoxyméthoxyméthyl)ptéridin-4(3H)-one

### a) 2-(Méthoxyméthoxy)acétimidate de méthyle

Obtenu en opérant comme dans le paragraphe a de l'exemple 2, à partir de 286,8 g (2,84 moles) de méthoxyméthoxyacétonitrile (préparé selon D.J. Loder et W.M. Bruner, brevet U.S. 2.398.757 ; C.A. 1946, 40, 3774) et de 6,5 g (0,284 atome-gramme) de sodium dans 1430 ml de méthanol. Durée de la réaction : 2 jours. Rdt : 302,6 g (80 %), Eb₁₅ = 62 - 65°C.
R.M.N. (CDCl₃) : δ = 3,3 (3H,s) ; 3,7 (3H,s) ; 3,9 (2H,s) ; 4,6 (2H,s) ; 7,7 (1H,pic échangeable avec D₂O).

### b) 2-(Méthoxyméthoxy)acétamidine

Obtenue en opérant comme dans le paragraphe b de l'exemple 2, à partir de 20,0 g (0,15 mole) de 2-(méthoxyméthoxy)acétimidate de méthyle et de 13,0 g (0,75 mole) d'ammoniac dans 400 ml d'éthanol absolu. Durée de la réaction : 2 jours. Rdt : 18,0 g (quantitatif).
R.M.N. (CDCl₃ + D₂O) : δ = 3,4 (3H,s) ; 4,0 (2H,s) ; 4,6 (2H,s).

### c) 4-Amino-2-(méthoxyméthoxyméthyl)ptéridine

Obtenue en opérant comme dans le paragraphe c de l'exemple 2, à partir de 12,0 g (0,10 mole) de 3-aminopyrazine-2-carbonitrile et de 18,0 g (0,15 mole) de 2-(méthoxyméthoxy)acétamidine dans 400 ml d'éthanol absolu. Durée du reflux : 4 heures. Rdt : 15,8 g (71 %), F = 129 - 131°C (éthanol).

| Analyse centésimale : C₉H₁₁N₅O₂ (M = 221,22) | | | |
|---|---|---|---|
| | C % | H % | N % |
| calculé | 48,86 | 5,01 | 31,66 |
| trouvé | 49,02 | 5,20 | 31,61 |

R.M.N. (DMSO d₆) : δ = 3,3 (3H,s) ; 4,5 (2H,s) ; 4,7 (2H,s) ; 8,2 (2H,pic échangeable avec CF₃COOD) ; 8,7 (1H,d) ; 8,9 (1H,d).

### d) 2-(Méthoxyméthoxyméthyl)ptéridin-4(3H)-one

Obtenue en opérant comme dans le paragraphe d de l'exemple 2, à partir de 5,3 g (0,024 mole) de 4-amino-2-(méthoxyméthoxyméthyl)ptéridine dans 250 ml de soude 5 %. Durée du chauffage : 2 heures à 80°C. Rdt : 2,2 g (41 %), F = 161 - 163°C (éthanol).

| Analyse centésimale : C₉H₁₀N₄O₃ (M = 222,20) | | | |
|---|---|---|---|
| | C % | H % | N % |
| calculé | 48,65 | 4,54 | 25,21 |
| trouvé | 48,38 | 4,52 | 24,92 |

IR : ν̅ (C = O) = 1680 cm⁻¹
R.M.N. (DMSO d₆) : δ = 3,2 (3H,s) ; 4,4 (2H,s) ; 4,7 (2H,s) ; 8,7 (1H,d) ; 8,9 (1H,d) ; 12,6 (1H,pic échangeable avec CF₃COOD).

### Exemple 13 :

### 2-(Isopropoxyméthyl)ptéridin-4(3H)-one

### a) 2-Isopropoxyacétamidine

Obtenue en opérant comme dans le paragraphe b de l'exemple 2, à partir de 14,7 g (0,112 mole) de 2-isopropoxyacétimidate de méthyle (préparé selon F.C. Schaefer et G.A. Peters, J. Org. Chem. 1961, 26, 412) et de 9,5 g (0,56 mole) d'ammoniac dans 300 ml d'éthanol absolu. Durée de la réaction : 2 jours. Rdt : 13,0 g (quantitatif).

### b) 4-Amino-2-(isopropoxyméthyl)ptéridine

Obtenue en opérant comme dans le paragraphe c de l'exemple 2, à partir de 8,8 g (0,073 mole) de 3-aminopyrazine-2-carbonitrile et de 13,0 g (0,11 mole) de 2-isopropoxyacétamidine dans 295 ml d'éthanol absolu. Durée du reflux : 4 heures. Rdt : 4,5 g (28 %), F = 139 - 141°C (acétate d'éthyle).

| Analyse centésimale : C₁₀H₁₃N₅O (M = 219,25) | | | |
|---|---|---|---|
| | C % | H % | N % |
| calculé | 54,78 | 5,98 | 31,94 |
| trouvé | 54,88 | 6,17 | 31,83 |

R.M.N. (CDCl₃) : δ = 1,3 (6H,d) ; 3,8 (1H,septuplet) ; 4,7 (2H,s) ; 8,2 (2H,pic échangeable avec CF₃COOD) ; 8,6 (1H,d) ; 9,0 (1H,d).

### c) 2-(Isopropoxyméthyl)ptéridin-4(3H)-one

Obtenue en opérant comme dans le paragraphe d de l'exemple 2, à partir de 4,5 g (0,0205 mole) de 4-amino-2-(isopropoxyméthyl)ptéridine dans 150 ml de soude 5 %. Durée du chauffage : 2 heures à 80°C. Rdt : 3,0 g (66 %), F = 198 - 199°C (éthanol).

| Analyse centésimale : C₁₀H₁₂N₄O₂ (M = 220,23) | | | |
|---|---|---|---|
| | C % | H % | N % |
| calculé | 54,54 | 5,49 | 25,44 |
| trouvé | 54,24 | 5,20 | 25,29 |

IR : ν̅ (C = O) = 1690 cm⁻¹
R.M.N. (CDCl₃) : δ = 1,3 (6H,d) ; 3,8 (1H,septuplet) ; 4,6 (2H,s) ; 8,7 (1H,d) ; 8,9 (1H,d) ; 10,0 (1H,pic échangeable avec CF₃COOD).

### Exemple 14 :

### 2-[(2,2,2-Trifluoroéthoxy)méthyl]ptéridin-4(3H)-one

### a) (2,2,2-Trifluoroéthoxy)acétonitrile

Vers 50°C, 26,4 g (0,40 mole) de potasse 85 % sont solubilisés dans 100 g (1,0 mole) de 2,2,2-trifluoroéthanol. Après refroidissement à 25°C, 37,8 g (0,50 mole) de chloroacétonitrile sont ajoutés goutte à goutte à cette solution. La température s'élève lentement jusqu'à 40°C et un précipité apparaît. Le milieu réactionnel est alors porté progressivement à 60°C ; après adjonction de 300 ml d'heptane, il est ensuite porté à reflux. Toutes les vapeurs sont condensées au moyen d'un appareil de Dean-Stark. La phase inférieure du condensat est séparée par décantation et distillée sous pression atmosphérique. Rdt : 19,6 g (28 %), Eb = 125 - 140°C.
R.M.N. (CDCl₃) : δ = 3,9 (2H,q) ; 4,4 (2H,s).

### b) 2-(2,2,2-Trifluoroéthoxy)acétimidate de méthyle

Obtenu en opérant comme dans le paragraphe a de l'exemple 2, à partir de 19,6 g (0,141 mole) de (2,2,2-trifluoroéthoxy) acétonitrile et de 0,3 g (0,013 atome-gramme) de sodium dans 95 ml de méthanol. Durée de la réaction : 24 heures. Rdt : 15,6 g (65 %), Eb₁₅ = 44 - 47°C.
R.M.N. (CDCl₃) : δ = 3,8 (3H,s) ; 3,9 (2H,q) ; 4,0 (2H,s) ; 7,7 (1H,pic échangeable avec D₂O).

### c) 2-(2,2,2-Trifluoroéthoxy)acétamidine

Obtenue en opérant comme dans le paragraphe b de l'exemple 2, à partir de 15,6 g (0,091 mole) de 2-(2,2,2-trifluoroéthoxy)acétimidate de méthyle et de 7,7 g (0,455 mole) d'ammoniac dans 225 ml d'éthanol absolu. Durée de la réaction : 24 heures. Rdt : 14,2 g (quantitatif).

### d) 4-Amino-2-[(2,2,2-trifluoroéthoxy)méthyl]ptéridine

Obtenue en opérant comme dans le paragraphe c de l'exemple 2, à partir de 7,2 g (0,060 mole) de 3-aminopyrazine-2-carbonitrile et de 14,2 g (0,091 mole) de 2-(2,2,2-trifluoroéthoxy)acétamidine dans 230 ml d'éthanol absolu. Durée du reflux : 7 heures. Rdt : 6,5 g (42 %), F = 145 - 147°C (acétate d'éthyle).

| Analyse centésimale : C₉H₈F₃N₅O (M = 259,19) | | | | |
|---|---|---|---|---|
| | C % | H % | F % | N % |
| calculé | 41,71 | 3,11 | 21,99 | 27,02 |
| trouvé | 41,92 | 2,96 | 21,78 | 26,93 |

R.M.N. (DMSO d₆) : δ = 4,2 (2H,q) ; 4,6 (2H,s) ; 8,3 (2H,pic échangeable avec CF₃COOD) ; 8,7 (1H,m) ; 9,0 (1H,m).

### e) 2-[(2,2,2-Trifluoroéthoxy)méthyl]ptéridin-4(3H)-one

Obtenue en opérant comme dans le paragraphe d de l'exemple 2, à partir de 5,0 g (0,0193 mole) de 4-amino-2-[(2,2,2(trifluoroéthoxy)méthyl]ptéridine dans 105 ml de soude 5 %. Durée du chauffage : 1 heure 30 à 80°C. Rdt : 2,5 g (50 %), F = 165 - 167°C (acétate d'éthyle).

| Analyse centésimale : C₉H₇F₃N₄O₂ (M = 260,18) | | | | |
|---|---|---|---|---|
| | C % | H % | F % | N % |
| calculé | 41,55 | 2,71 | 21,91 | 21,53 |
| trouvé | 41,53 | 2,68 | 21,78 | 21,32 |

IR : ν̅ (C = O) = 1690 cm⁻¹
R.M.N. (DMSO d₆ + CF₃COOD) : δ = 4,2 (2H,q) ; 4,6 (2H,s) ; 8,7 (1H,d) ; 8,9 (1H,d).

### Exemple 15 :

### 2-[(4-Chlorophénoxy)méthyl]ptéridin-4(3H)-one

### a) 4-Amino-2-[(4-chlorophénoxy)méthyl]ptéridine

Obtenue en opérant comme dans le paragraphe c de l'exemple 2, à partir de 10,7 g (0,089 mole) de 3-aminopyrazine-2-carbonitrile et de 24,5 g (0,133 mole) de 2-(4-chlorophénoxy)acétamidine (préparée selon C. Djerassi et C.R. Scholz, brevet U.S. 2.517.468 ; C.A. 1951, 45, 661f) dans 320 ml d'éthanol absolu. Durée du reflux : 7 heures. Rdt : 21,5 g (84 %), F = 246 - 248°C (N,N-diméthylformamide).

| Analyse centésimale : C₁₃H₁₀ClN₅O (M = 287,71) | | | | |
|---|---|---|---|---|
| | C % | H % | Cl % | N % |
| calculé | 54,27 | 3,50 | 12,32 | 24,34 |
| trouvé | 54,25 | 3,50 | 12,37 | 24,33 |

R.M.N. (DMSO d₆) : δ = 5,1 (2H,s) ; 6,9 (2H,d) ; 7,3 (2H,d) ; 8,3 (2H,pic échangeable avec CF₃COOD) ; 8,7 (1H,d) ; 9,0 (1H,d).

### b) 2-[(4-Chlorophénoxy)méthyl]ptéridin-4(3H)-one

Obtenue en opérant comme dans le paragraphe b de l'exemple 9, à partir de 17,5 g (0,0608 mole) de 4-amino-2-[(4-chlorophénoxy)méthyl]ptéridine brute dans 1750 ml de soude 5 %. Durée du chauffage : 1 heure 30 à reflux. Rdt : 8,7 g (50 %), F = 267 - 269°C (éthanol - N,N-diméthylformamide).

| Analyse centésimale : C₁₃H₉ClN₄O₂ (M = 288,69) | | | | |
|---|---|---|---|---|
| | C % | H % | Cl % | N % |
| calculé | 54,09 | 3,14 | 12,28 | 19,41 |
| trouvé | 54,04 | 2,94 | 12,42 | 19,20 |

IR : ν̅ (C = O) = 1675 cm⁻¹
R.M.N. (CF₃COOD) : δ = 5,5 (2H,s) ; 7,1 (2H,d) ; 7,4 (2H,d) ; 9,2 (1H,d) ; 9,3 (1H,d).

### Exemple 16 :

### 2-[(3,4-Dichlorophénoxy)méthyl]ptéridin-4(3H)-one

### a) 2-(3,4-Dichlorophénoxy)acétimidate de méthyle

On ajoute 30,3 g (0,150 mole) de (3,4-dichlorophénoxy)acétonitrile (préparé selon R.W. Fuller et al., J. Med. Chem. 1973, 16, 101) à une solution de méthylate de sodium dans le méthanol, obtenue par réaction de 0,34 g (0,015 atome-gramme) de sodium dans 300 ml de méthanol. Le milieu réactionnel est agité pendant 36 heures à température ambiante. Le méthylate de sodium est alors neutralisé par un courant de gaz carbonique, puis le milieu réactionnel est concentré à sec sous pression réduite. Le résidu obtenu est repris au chlorure de méthylène. Les produits minéraux sont éliminés par filtration ; le filtrat organique est concentré à sec sous pression réduite et le résidu est recristallisé dans l'éther isopropylique. Rdt : 28,2 g (80 %), F = 55 - 56°C.

### b) 2-(3,4-Dichlorophénoxy)acétamidine

Obtenue en opérant comme dans le paragraphe b de l'exemple 2, à partir de 28,2 g (0,12 mole) de 2-(3,4-dichlorophénoxy)acétamidate de méthyle et de 10,2 g (0,60 mole) d'ammoniac dans 480 ml d'éthanol absolu. Durée de la réaction : 3 jours. Rdt : 26,3 g (quantitatif).

### c) 4-Amino-2-[(3,4-dichlorophénoxy)méthyl]ptéridine

Obtenue en opérant comme dans le paragraphe c de l'exemple 2, à partir de 9,6 g (0,080 mole) de 3-aminopyrazine-2-carbonitrile et de 26,3 g (0,12 mole) de 2-(3,4-dichlorophénoxy)acétamidine dans 520 ml d'éthanol absolu. Durée du reflux : 3 heures. Rdt : 18,0 g (70 %), F = 261 - 263°C (éthanol - N,N-diméthylformamide).

| Analyse centésimale : C₁₃H₉Cl₂N₅O (M = 322,15) | | | | |
|---|---|---|---|---|
| | C % | H % | Cl % | N % |
| calculé | 48,47 | 2,82 | 22,01 | 21,74 |
| trouvé | 48,50 | 2,85 | 22,31 | 21,65 |

R.M.N. (CF₃COOD) : δ = 5,4 (2H,s) ; 6,9 - 7,6 (3H,m) ; 9,2 (2H,s).

### d) 2-[(3,4-Dichlorophénoxy)méthyl]ptéridin-4(3H)-one

Obtenue en opérant comme dans le paragraphe b de l'exemple 9, à partir de 15,0 g (0,0466 mole) de 4-amino-2-[(3,4-dichlorophénoxy)méthyl]ptéridine dans 1500 ml de soude 5 %. Durée du chauffage : 6 heures à reflux. Rdt : 10,0 g (66 %), F = 285 - 287°C (éthanol - N,N-diméthylformamide).

| Analyse centésimale : C₁₃H₈Cl₂N₄O₂ (M = 323,14) | | | | |
|---|---|---|---|---|
| | C % | H % | Cl % | N % |
| calculé | 48,32 | 2,50 | 21,94 | 17,34 |
| trouvé | 48,55 | 2,51 | 21,91 | 16,89 |

IR : ν̅ (C = O) = 1690 cm⁻¹
R.M.N. (CF₃COOD) : δ = 5,6 (2H,s) ; 7,0 - 7,7 (3H,m) ; 9,3 (1H,d) ; 9,4 (1H,d).

### Exemple 17 :

### 2-[(4-Méthoxyphénoxy)méthyl]ptéridin-4(3H)-one

### a) 2-(4-Méthoxyphénoxy)acétimidate de méthyle

Obtenu en opérant comme dans le paragraphe a de l'exemple 16, à partir de 27,3 g (0,167 mole) de (4-méthoxyphénoxy)acétonitrile (préparé selon K.J.S. Arora et al., J. Chem. Soc. C 1971, 2865) et de 0,38 g (0,0167 atome-gramme) de sodium dans 275 ml de méthanol. Durée de la réaction : 24 heures. Rdt : 23,9 g (73 %), F = 95 - 96°C (éther isopropylique).

### b) 2-(4-Méthoxyphénoxy)acétamidine

A une solution, maintenue à 10°C, de 10,4 g (0,61 mole) d'ammoniac dans 480 ml d'éthanol absolu, on ajoute 23,9 g (0,122 mole) de 2-(4-méthoxyphénoxy)acétimidate de méthyle. Le milieu réactionnel est abandonné pendant 2 jours à température ambiante, puis l'excès d'ammoniac est chassé par un courant d'azote. La solution obtenue est utilisée dans l'étape suivante sans autre purification.

### c) 4-Amino-2-[(4-méthoxyphénoxy)méthyl]ptéridine

Obtenue en opérant comme dans le paragraphe c de l'exemple 2, à partir de 9,8 g (0,082 mole) de 3-aminopyrazine-2-carbonitrile et de la solution précédente de 2-(4-méthoxyphénoxy)acétamidine dans l'éthanol absolu. Durée du reflux : 2 heures. Rdt : 5,5 g (24 %), F = 238 - 240°C (éthanol - N,N-diméthylformamide).

| Analyse centésimale : C₁₄H₁₃N₅O₂ (M = 283,29) | | | |
|---|---|---|---|
| | C % | H % | N % |
| çalculé | 59,36 | 4,63 | 24,72 |
| trouvé | 59,21 | 4,62 | 24,71 |

R.M.N. (DMSO d₆) : δ = 3,7 (3H,s) ; 5,0 (2H,s) ; 6,9 (4H,s) ; 8,3 (2H,pic échangeable avec CF₃COOD) ; 8,8 (1H,d) ; 9,0 (1H,d).

### d) 2-[(4-Méthoxyphénoxy)méthyl]ptéridin-4(3H)-one

Obtenue en opérant comme dans le paragraphe b de l'exemple 9, à partir de 5,5 g (0,0194 mole) de 4-amino-2-[(4-méthoxyphénoxy) méthyl]ptéridine dans 150 ml de soude 5 %. Le solide obtenu est repris dans l'eau sodée pour purification. La partie insoluble est éliminée par filtration. Le filtrat est acidifié par l'acide acétique jusqu'à un pH égal à 5,5. Le précipité formé est isolé par filtration ; il est enfin recristallisé dans un mélange d'éthanol et de N,N-diméthylformamide. Rdt : 1,7 g (31 %), F = 233 - 234°C.

| Analyse centésimale : C₁₄H₁₂N₄O₃ (M = 284,27) | | | |
|---|---|---|---|
| | C % | H % | N % |
| calculé | 59,15 | 4,26 | 19,71 |
| trouvé | 59,21 | 4,34 | 19,39 |

IR : ν̅ (C = O) = 1690 cm⁻¹
R.M.N. (DMSO d₆) : δ = 3,7 (3H,s) ; 5,0 (2H,s) ; 6,8 (2H,d) ; 7,0 (2H,d) ; 8,8 (1H,d); 9,0 (1H,d) ; 13,0 (1H,pic échangeable avec CF₃COOD).

### Exemple 18 :

### 2-[(4-Méthylphénoxy)méthyl)]ptéridin-4(3H)-one

### a) 4-Amino-2-[(4-méthylphénoxy)méthyl]ptéridine

Obtenue en opérant comme dans le paragraphe c de l'exemple 2, à partir de 6,9 g (0,057 mole) de 3-aminopyrazine-2-carbonitrile et de 14,1 g (0,086 mole) de 2-(4-méthylphénoxy)acétamidine (préparée selon C. Djerassi et C.R. Scholz, brevet U.S. 2.517.468 ; C.A. 1951, 45, 661f) dans 350 ml d'éthanol absolu. Durée du reflux : 2 heures. Rdt : 2,6 g (17 %), F = 225 - 227°C (acétone-éthanol).

| Analyse centésimale : C₁₄H₁₃N₅O (M = 267,29) | | | |
|---|---|---|---|
| | C % | H % | N % |
| calculé | 62,91 | 4,90 | 26,20 |
| trouvé | 63,22 | 4,93 | 26,23 |

R.M.N. (DMSO d₆) : δ = 2,2 (3H,s) ; 5,0 (2H,s) ; 6,8 (2H,d) ; 7,0 (2H,d) ; 8,3 (2H,pic échangeable avec CF₃COOD) ; 8,8 (1H,d) ; 9,0 (1H,d).

### b) 2-[(4-Méthylphénoxy)méthyl]ptéridin-4(3H)-one

Obtenue en opérant comme dans le paragraphe b de l'exemple 9, à partir de 2,5 g (0,0094 mole) de 4-amino-2-[(4-méthylphénoxy)méthyl)]ptéridine dans 68,1 ml de soude 5 %. Durée du chauffage : 3 heures 15 à 85°C. Rdt : 1,3 g (52 %), F = 232 - 233°C (éthanol - N,N-diméthylformamide).

| Analyse centésimale : C₁₄H₁₂N₄O₂ (M = 268,28) | | | |
|---|---|---|---|
| | C % | H % | N % |
| calculé | 62,68 | 4,51 | 20,88 |
| trouvé | 62,89 | 4,54 | 21,29 |

IR : ν̅ (C = O) = 1690 cm⁻¹
R.M.N. (DMSO d₆) : δ = 2,2 (3H,s) ; 5,0 (2H,s) ; 6,9 (2H,d) ; 7,1 (2H,d) ; 8,8 (1H,d) ; 9,0 (1H,d) ; 12,9 (1H, pic échangeable avec CF₃COOD).

### Exemple 19 :

### 2-(Benzylthiométhyl)ptéridin-4(3H)-one

### a) 4-Amino-2-(benzylthiométhyl)ptéridine

Une suspension de 2,3 g (0,019 mole) de 3-aminopyrazine-2-carbonitrile et de 5,2 g (0,029 mole) de 2-(benzylthio)acétamidine (préparée selon J.M. Mc Manus, J. Heterocycl. Chem. 1968, 5, 137) dans 150 ml d'éthanol absolu est portée à reflux pendant 2 heures. Après refroidissement, la solution obtenue est concentrée à sec sous pression réduite. Le résidu est lavé à l'éther isopropylique et séché sous pression réduite. On obtient un solide amorphe que l'on utilise dans l'étape suivante sans autre purification. Rdt : 5,4 g (quantitatif).

### b) 2-(Benzylthiométhyl)ptéridin-4(3H)-one

Obtenue en opérant comme dans le paragraphe b de l'exemple 9, à partir de 5,4 g (0,019 mole) de 4-amino-2-(benzylthiométhyl)ptéridine dans 400 ml de soude 5 %. Durée de la réaction : 7 heures à 85°C. Rdt : 1,7 g (31 %), F = 224 - 226°C (éthanol - N,N-diméthylformamide).

| Analyse centésimale : C₁₄H₁₂N₄OS (M = 284,34) | | | | |
|---|---|---|---|---|
| | C % | H % | N % | S % |
| calculé | 59,14 | 4,25 | 19,70 | 11,28 |
| trouvé | 58,92 | 4,35 | 20,02 | 11,10 |

IR : ν̅ (C = O) = 1690 cm⁻¹
R.M.N. (DMSO d₆) : δ = 3,6 (2H,s échangeable avec CF₃COOD) ; 3,9 (2H,s) ; 7,0 - 7,5 (5H,m) ; 8,8 (1H,d) ; 9,0 (1H,d) ; 12,8 (1H,pic échangeable avec CF₃COOD).

### Exemple 20 :

### 2-(Ethoxyméthyl)-6-méthylptéridin-4(3H)-one

### a) 2-Ethoxyacétimidate d'éthyle

Une solution de 604 g (8,0 moles) de chloroacétonitrile dans 1200 ml d'éthanol absolu est ajoutée goutte à goutte à une solution, maintenue à 80°C, d'éthylate de sodium dans l'éthanol, obtenue à partir de 193,2 g (8,4 atomes-gramme) de sodium et de 4 l d'éthanol absolu. Le milieu réactionnel est laissé revenir à température ambiante ; l'agitation est poursuivie pendant 2 jours. L'excès d'éthylate de sodium est ensuite neutralisé par un courant de gaz carbonique. Le précipité formé est éliminé par filtration et lavé à l'éther. Le filtrat et la solution éthérée de lavage sont rassemblés et concentrés sous pression réduite. Il se forme un nouveau précipité qui est éliminé par filtration. Le filtrat est distillé sous pression réduite. Rdt : 597,6 g (57 %), Eb₁₅₋₂₄ = 47 - 52°C.
R.M.N. (CDCl₃ + CF₃COOD) : δ = 1,1 (3H,t) ; 1,2 (3H,t) ; 3,4 (2H,q) ; 3,7 (2H,s) ; 4,1 (2H,q).

### b) 2-Ethoxyacétamidine

Obtenue en opérant comme dans le paragraphe b de l'exemple 17, à partir de 29,4 g (0,224 mole) de 2-éthoxyacétimidate d'éthyle et de 21,2 g (1,24 mole) d'ammoniac dans 590 ml d'éthanol absolu.

### c) 4-Amino-2-(éthoxyméthyl)-6-méthylptéridine

Une suspension de 15,1 g (0,113 mole) de 3-amino-6-méthylpyrazine-2-carbonitrile (préparé selon E.C. Taylor et al., J. Am. Chem. Soc. 1973, 95, 6413) dans la solution éthanolique précédente de 2-éthoxyacétamidine est portée à reflux pendant 2 heures. Après refroidissement, la solution obtenue est concentrée à sec sous pression réduite. Le résidu est purifié par lavage à l'acétone et recristallisation dans un mélange d'acétone et d'éther isopropylique. Rdt : 8,4 g (34 %), F = 166 - 168°C.

| Analyse centésimale : C₁₀H₁₃N₅O (M = 219,25) | | | |
|---|---|---|---|
| | C % | H % | N % |
| calculé | 54,78 | 5,98 | 31,94 |
| trouvé | 54,63 | 6,02 | 32,10 |

R.M.N. (DMSO d₆) : δ = 1,1 (3H,t) ; 2,6 (3H,s) ; 3,6 (2H,q) ; 4,4 (2H,s) ; 8,0 (2H,pic échangeable avec CF₃COOD) ; 8,9 (1H,s).

### d) 2-(Ethoxyméthyl)-6-méthylptéridin-4(3H)-one

Une suspension de 7,4 g (0,0338 mole) de 4-amino-2-(éthoxyméthyl)-6-méthylptéridine dans 148 ml de soude 5 % est portée lentement à 85°C et maintenue à cette température pendant 2 heures. On rajoute ensuite 592 ml de soude 5 % et on poursuit le chauffage à 85°C pendant 1 heure. Après refroidissement, la solution obtenue est acidifiée par l'acide acétique jusqu'à un pH égal à 5,5, puis est extraite au chlorure de méthylène. Les extraits organiques sont séchés sur sulfate de sodium et concentrés à sec sous pression réduite. Le résidu solide est recristallisé dans l'acétone en présence de Norit. Rdt : 4,9 g (66 %), F = 181 - 183°C.

| Analyse centésimale : C₁₀H₁₂N₄O₂ (M = 220,23) | | | |
|---|---|---|---|
| | C % | H % | N % |
| calculé | 54,54 | 5,49 | 25,44 |
| trouvé | 54,39 | 5,49 | 25,22 |

IR : ν̅ (C = O) = 1670 cm⁻¹
R.M.N. (DMSO d₆) : δ = 1,2 (3H,t) ; 2,6 (3H,s) ; 3,6 (2H,q) ; 4,4 (2H,s) ; 8,8 (1H,s) ; 13,8 (1H,pic échangeable avec CF₃COOD).

### Exemple 21 :

### 2-[(2-Acétoxyéthoxy)méthyl]ptéridin-4(3H)-one

### a) 2-(2-Hydroxyéthoxy)acétimidate d'éthyle

On additionne 55,7 g (0,389 mole) de (2-acétoxyéthoxy)acétonitrile (préparé selon S.W. Schneller et al., Croat. Chem. Acta 1986, 59, 307) à une solution, maintenue à 10°C, d'éthylate de sodium dans l'éthanol, obtenue à partir de 0,89 g (0,0389 atome-gramme) de sodium et de 560 ml d'éthanol absolu. Le milieu réactionnel est laissé revenir à température ambiante ; l'agitation est poursuivie pendant 3 jours. L'excès d'éthylate de sodium est ensuite neutralisé par un courant de gaz carbonique. Le précipité formé est éliminé par filtration et lavé à l'éther. Le filtrat et la solution éthérée de lavage sont rassemblés et concentrés sous pression réduite à 30°C. Le résidu obtenu est repris par 800 ml d'éther. Par refroidissement, il se forme un nouveau précipité qui est éliminé par filtration. Le filtrat est concentré sous pression réduite à une température inférieure à 30°C. Le liquide obtenu est utilisé dans l'étape suivante sans autre purification. Rdt : 39,2 g (68 %).
R.M.N. (CDCl₃ + D₂O) : δ = 1,3 (3H,t) ; 3,3 - 3,9 (4H,m) ; 3,9 (2H,s) ; 4,2 (2H,q).

### b) 2-(2-Hydroxyéthoxy)acétamidine

Obtenue en opérant comme dans le paragraphe b de l'exemple 17, à partir de 39,2 g (0,266 mole) de 2-(2-hydroxyéthoxy)acétimidate d'éthyle et de 22,6 g (1,33 mole) d'ammoniac dans 1080 ml d'éthanol absolu.

### c) 4-Amino-2-[(2-hydroxyéthoxy)méthyl]ptéridine

Obtenue en opérant comme dans le paragraphe c de l'exemple 2, à partir de 16,0 g (0,133 mole) de 3-aminopyrazine-2-carbonitrile et de la solution précédente de 2-(2-hydroxyéthoxy)acétamidine dans l'éthanol absolu. Durée du reflux : 2 heures 30. Rdt : 20,5 g (70 %), F = 149 - 151°C (éthanol). Un échantillon analytique a été obtenu par recristallisation dans un mélange d'acétone et d'éthanol. F = 159 - 161°C.

| Analyse centésimale : C₉H₁₁N₅O₂ (M = 221,22) | | | |
|---|---|---|---|
| | C % | H % | N % |
| calculé | 48,86 | 5,01 | 31,66 |
| trouvé | 48,83 | 5,02 | 31,32 |

R.M.N. (DMSO d₆) : δ = 3,6 (4H,s) ; 4,5 (2H,s) ; 4,9 (1H,pic échangeable avec CF₃COOD) ; 8,3 (2H,pic échangeable avec CF₃COOD) ; 8,8 (1H,d) ; 9,0 (1H,d).

### d) 2-[(2-Acétoxyéthoxy)méthyl]ptéridin-4(3H)-one

Une solution de 10,0 g (0,0452 mole) de 4-amino-2-[(2-hydroxyéthoxy)méthyl]ptéridine dans 350 ml de soude 5 % est portée progressivement à 85°C et maintenue à cette température pendant 1 heure 30. Après refroidissement, la solution obtenue est acidifiée par l'acide acétique jusqu'à un pH égal à 5,5, puis est concentrée à sec sous pression réduite. Le résidu est repris dans 500 ml d'isopropanol. La partie minérale insoluble est éliminée par filtration. La solution isopropanolique est concentrée à sec sous pression réduite. Le résidu solide est additionné de 250 ml d'anhydride acétique et ce mélange est porté à reflux pendant 1 heure. Après refroidissement, des sels minéraux sont éliminés par filtration ; le filtrat est concentré à sec sous pression réduite. Le résidu est purifié par 4 recristallisations successives dans l'éthanol en présence de Norit. Rdt : 1,2 g (10 %). F = 157 - 159°C.

| Analyse centésimale : C₁₁H₁₂N₄O₄ (M = 264,24) | | | |
|---|---|---|---|
| | C % | H % | N % |
| calculé | 50,00 | 4,58 | 21,20 |
| trouvé | 50,05 | 4,75 | 21,08 |

IR : ν̅ (C = O) = 1690 et 1720 cm⁻¹
R.M.N. (DMSO d₆) : δ = 2,0 (3H,s) ; 3,7 - 4,0 (2H,m) ; 4,1 - 4,4 (2H,m) ; 4,5 (2H,s) ; 8,8 (1H,d) ; 9,0 (1H,d) ; 12,6 (1H,pic échangeable avec CF₃COOD).

### Exemple 22 :

### 2-[(4-Acétyl-3-hydroxy-2-propylphénoxy)méthyl]ptéridin-4(3H)-one

### a) Chlorhydrate de 2-(4-acétyl-3-hydroxy-2-propylphénoxy)acétimidate d'éthyle

Obtenu en opérant comme dans le paragraphe a de l'exemple 6, à partir de 30,4 g (0,130 mole) de (4-acétyl-3-hydroxy-2-propylphénoxy)acétonitrile (préparé selon W.S. Marshall et al., J. Med. Chem. 1987, 30, 682) et de 6,6 g (0,143 mole) d'éthanol absolu dans 500 ml d'éther. Durée de la réaction : 16 heures. Après avoir jeté le milieu réactionnel dans 1 l d'éther, le produit de la réaction est isolé par filtration. Rdt. : 40,0 g (97 %), F = 157 - 159°C.
R.M.N. (DMSO d₆) : δ = 0,9 (3H,t) ; 1,1 - 1,9 (2H,m) ; 1,4 (3H,t) ; 2,4 - 2,9 (2H,m) ; 2,6 (3H,s) ; 4,6 (2H,q) ; 5,3 (2H,s) ; 6,7 (1H,d) ; 7,8 (1H,d) ; 10,8 (2H,pic échangeable avec CF₃COOD) ; 12,8 (1H,s échangeable avec CF₃COOD).

### b) 2-(4-Acétyl-3-hydroxy-2-propylphénoxy)acétimidate d'éthyle

A une suspension de 40,0 g (0,127 mole) de chlorhydrate de 2-(4-acétyl-3-hydroxy-2-propylphénoxy)acétimidate d'éthyle dans 1 l de chloroforme, on ajoute 10,2 g (0,121 mole) de bicarbonate de sodium et on agite ce mélange pendant 1 heure. Le solide est éliminé par filtration et le filtrat est concentré à sec sous pression réduite. Le résidu obtenu est extrait par un mélange de 100 ml d'hexane et de 100 ml d'éther isopropylique à reflux. Cet extrait, concentré sous pression réduite, est recristallisé dans l'hexane. Rdt : 25,0 g (70 %), F = 50 - 52°C.
R.M.N. (CDCl₃ + D₂O) : δ = 1,0 (3H,t) ; 1,1 - 1,9 (2H,m) ; 1,4 (3H,t) ; 2,6 (3H,s) ; 2,7 (2H,t) ; 4,3 (2H,q) ; 4,7 (2H,s) ; 6,3 (1H,d) ; 7,6 (1H,d) ; 12,7 (1H,s partiellement échangé).

### c) 2-(4-Acétyl-3-hydroxy-2-propylphénoxy)acétamidine

Obtenue en opérant comme dans le paragraphe b de l'exemple 17, à partir de 25,0 g (0,0895 mole) de 2-(4-acétyl-3-hydroxy-2-propylphénoxy)acétimidate d'éthyle et de 7,6 g (0,45 mole) d'ammoniac dans 300 ml d'éthanol absolu.

### d) 2-[(4-Acétyl-3-hydroxy-2-propylphénoxy)méthyl]-4-aminoptéridine

Obtenue en opérant comme dans le paragraphe c de l'exemple 2, à partir de 3,6 g (0,030 mole) de 3-aminopyrazine-2-carbonitrile et de la solution précédente de 2-(4-acétyl-3-hydroxy-2-propylphénoxy)acétamidine dans l'éthanol absolu. Durée du reflux : 6 heures. Rdt : 3,2 g (30 %), F = 178 - 180°C (éthanol - N,N-diméthylformamide).
Un échantillon analytique a été obtenu par une recristallisation supplémentaire dans un mélange d'éthanol et de N,N-diméthylformamide. F = 179 - 181°C.

| Analyse centésimale : C₁₈H₁₉N₅O₃ (M = 353,38) | | | |
|---|---|---|---|
| | C % | H % | N % |
| calculé | 61,18 | 5,42 | 19,82 |
| trouvé | 60,96 | 5,59 | 19,99 |

IR : ν̅ (C = O) = 1640 cm⁻¹
R.M.N. (DMSO d₆) : δ = 0,9 (3H,t) ; 1,1 - 1,9 (2H,m) ; 2,5 (3H,s) ; 2,5 - 2,9 (2H,m) ; 5,2 (2H,s) ; 6,6 (1H,d) ; 7,7 (1H,d) ; 8,3 (2H,pic échangeable avec CF₃COOD) ; 8,8 (1H,d) ; 9,0 (1H,d) ; 13,9 (1H,pic échangeable avec CF₃COOD).

### e) 2-[(4-Acétyl-2-hydroxy-3-propylphénoxy)méthyl]ptéridin-4(3H)-one

Obtenue en opérant comme dans le paragraphe d de l'exemple 2, à partir de 1,6 g (0,0045 mole) de 2-[(4-acétyl-3-hydroxy-2-propylphénoxy)méthyl]-4-aminoptéridine dans 32 ml de soude 5 %. Durée du chauffage : 1 heure à 90°C. Rdt : 1,0 g (63 %), F = 224 - 226°C (éthanol - N,N-diméthylformamide).

| Analyse centésimale : C₁₈H₁₈N₄O₄ (M = 354,37) | | | |
|---|---|---|---|
| | C % | H % | N % |
| calculé | 61,01 | 5,12 | 15,81 |
| trouvé | 61,12 | 5,23 | 15,89 |

IR : ν̅ (C = O) = 1670 et 1690 cm⁻¹
R.M.N. (DMSO d₆) : δ = 0,9 (3H,t) ; 1,0 - 2,0 (2H,m) ; 2,4 - 2,9 (2H,m) ; 2,5 (3H,s) ; 5,2 (2H,s) ; 6,7 (1H,d) ; 7,7 (1H,d) ; 8,8 (1H,d) ; 8,9 (1H,d) ; 12,8 (1H,s échangeable avec CF₃COOD) ; 13,0 (1H,pic échangeable avec CF₃COOD).

### Exemple 23 :

### 7-Hydroxy-2-(hydroxyméthyl)ptéridin-4(3H)-one

Un mélange de 4,0 g (0,0169 mole) de 2-(acétoxyméthyl)-7-hydroxyptéridin-4(3H)-one, de 1,2 g (0,0214 mole) de potasse et de 5 ml d'eau dans 80ml d'éthanol est porté à reflux pendant 4 heures. Après refroidissement, le milieu réactionnel est filtré. Le solide ainsi isolé, est lavé par 30 ml d'éthanol et repris dans 160 ml d'eau. La solution trouble obtenue est clarifiée par filtration et acidifiée par l'acide acétique jusqu'à un pH égal à 3,6. Le précipité formé est isolé par filtration. Il est purifié par lavage à l'éther et recristallisation dans un mélange d'éthanol et de N,N-diméthylformamide. Rdt : 2,0 g (61 %), F > 300°C.

| Analyse centésimale : C₇H₆N₄O₃ (M = 194,15) | | | |
|---|---|---|---|
| | C % | H % | N % |
| calculé | 43,31 | 3,12 | 28,86 |
| trouvé | 43,27 | 3,37 | 28,92 |

IR : ν̅ (C = O) = 1640 et 1675 cm⁻¹
R.M.N. (DMSO d₆ + CF₃COOD) : δ = 4,4 (2H,s) ; 7,9 (1H,s).

### Exemple 24 :

### 7-Hydroxy-2-(méthoxyméthoxyméthyl)ptéridin-4(3H)-one

### a) 2-(Méthoxyméthoxy)acétamidine

Obtenue en opérant comme dans le paragraphe b de l'exemple 17, à partir de 14,4 g (0,108 mole) de 2-(méthoxyméthoxy)acétimidate de méthyle et de 7,6 g (0,446 mole) d'ammoniac dans 146 ml de méthanol anhydre. Durée de la réaction : 3 jours.

### b) 4-Amino-7-méthoxy-2-(méthoxyméthoxyméthyl)ptéridine

Obtenue en opérant comme dans le paragraphe c de l'exemple 2, à partir de 11,2 g (0,0725 mole) de 3-amino-5-chloropyrazine-2-carbonitrile (préparé selon E.C. Taylor et al. 1975, 40, 2341) et de la solution précédente de 2-(méthoxyméthoxy)acétamidine dans le méthanol. Durée du reflux : 5 heures. Rdt : 13,2 g (72 %), F = 193 - 195°C.
R.M.N. (DMSO d₆) : δ = 3,3 (3H,s) ; 4,0 (3H,s) ; 4,5 (2H,s) ; 4,7 (2H,s) ; 7,9 (2H,pic échangeable avec CF₃COOD) ; 8,4 (1H,s).

### c) 4-Amino-7-hydroxy-2-(méthoxyméthoxyméthyl)ptéridine

A une solution de 3,2 g (0,080 mole) de soude dans 130 ml d'eau, on ajoute 8,1 g (0,0322 mole) de 4-amino-7-méthoxy-2-(méthoxyméthoxyméthyl)ptéridine. La suspension obtenue est portée à 80°C pendant 2 heures. Après filtration à chaud, la solution est laissée revenir à température ambiante, puis elle est acidifiée par l'acide acétique. Le précipité formé est isolé par filtration. Il est purifié par lavage à l'eau, puis à l'acétone et est recristallisé dans un mélange de méthanol et de N,N-diméthylformamide. Rdt : 4,4 g (58 %), F = 210 - 212°C.

| Analyse centésimale : C₉H₁₁N₅O₃ (M = 237,22) | | | |
|---|---|---|---|
| | C % | H % | N % |
| calculé | 45,57 | 4,67 | 29,52 |
| trouvé | 45,30 | 4,53 | 29,56 |

R.M.N. (DMSO d₆) : δ = 3,3 (3H,s) ; 4,4 (2H,s) ; 4,7 (2H,s) ; 7,7 (2H,pic échangeable avec CF₃COOD) ; 7,9 (1H,s) ; 12,7 (1H,pic échangeable avec CF₃COOD).

### d) 7-Hydroxy-2-(méthoxyméthoxyméthyl)ptéridin-4(3H)-one

Dans une solution de 1,5 g (0,0267 mole) de potasse dans 20 ml d'eau, on ajoute 1,1 g (0,00464 mole) de 4-amino-7-hydroxy-2-(méthoxyméthoxyméthyl)ptéridine. Le mélange est porté à reflux pendant 8 heures. Après refroidissement, le milieu réactionnel est acidifié par de l'acide chlorhydrique 10 % jusqu'à un pH égal à 5,5. Le précipité formé est isolé par filtration et lavé à l'eau. Il est extrait par un mélange de 50 ml d'éthanol et de 60 ml de N,N-diméthylformamnide à reflux. Cet extrait, concentré sous pression réduite, est recristallisé dans un mélange d'éthanol et de N,N-diméthylformamide. Rdt : 0,2 g (18 %), F = 224 - 226°C (éthanol).

| Analyse centésimale : C₉H₁₀N₄O₄ (M = 238,20) | | | |
|---|---|---|---|
| | C % | H % | N % |
| calculé | 45,38 | 4,23 | 23,52 |
| trouvé | 45,15 | 4,11 | 23,59 |

IR : ν̅ (C = O) = 1625 cm⁻¹
R.M.N. (DMSO d₆) : δ = 3,3 (3H,s) ; 4,4 (2H,s) ; 4,7 (2H,s) ; 7,9 (1H,s) ; 12,8 (2H,pic échangeable avec CF₃COOD).

### Exemple 25 :

### 2-(Ethoxyméthyl)ptéridin-4(3H)-one

### a) 6-Amino-2-(éthoxyméthyl)pyrimidin-4(3H)-one

Dans une solution, maintenue à 0°C, de 75,7 g (0,577 mole) de 2-éthoxyacétimidate d'éthyle dans 650 ml d'éthanol absolu, on fait barboter un courant d'ammoniac, de façon à en dissoudre 39,1 g (2,30 moles). La solution obtenue est laissée sous agitation à température ambiante pendant 2 jours. L'excès d'ammoniac est ensuite chassé par un courant d'azote. Le milieu réactionnel est additionné de 75,0 g (0,383 mole) de chlorhydrate de (1-éthoxyformimidoyl)acétate d'éthyle (préparé selon J.J. Ursprung, brevet U.S. 3.337.579 ; C.A. 1968, 68, 68986k), puis vers 0°C, de 27,3 g (0,401 mole) d'éthylate de sodium. Après 15 minutes à 0°C, le solide en suspension est éliminé par filtration ; le filtrat est agité pendant 24 heures à température ambiante, puis est porté à reflux pendant 2 heures. Cette solution est concentrée par l'élimination, au moyen d'un appareil de Dean-Stark, de 425 ml d'éthanol ; elle est ensuite refroidie vers -20°C. Le précipité formé est isolé par filtration, lavé à l'éther éthylique. Le solide obtenu est séché et utilisé dans l'étape suivante sans autre purification. Rdt : 39,5 g (61 %), F = 224 - 226°C. Un échantillon analytique a été obtenu par recristallisation dans un mélange d'isopropanol et d'éther isopropylique. F = 228°C.

| Analyse centésimale : C₇H₁₁N₃O₂ (M = 169,18) | | | |
|---|---|---|---|
| | C % | H % | N % |
| calculé | 49,70 | 6,55 | 24,84 |
| trouvé | 49,90 | 6,21 | 24,59 |

IR : ν̅ (C = O) = 1610 cm⁻¹
R.M.N. (DMSO d₆) : δ = 1,1 (3H,t) ; 3,5 (2H,q) ; 4,1 (2H,s) ; 4,9 (1H,s) ; 6,4 (2H,s échangeable avec CF₃COOD) ; 11,2 (1H,s échangeable avec CF₃COOD).

### b) 6-Amino-2-(éthoxyméthyl)-5-nitrosopyrimidin-4(3H)-one

On ajoute 17,7 g (0,256 mole) de nitrite de sodium, à une solution de 39,5 g (0,233 mole) de 6-amino-2-(éthoxyméthyl)pyrimidin-4(3H)-one dans 336 ml (0,336 mole) de soude 1N. Puis entre 0 et 5°C, on ajoute goutte à goutte une solution de 17,9 ml (0,320 mode) d'acide sulfurique 96 % dilués dans 179 ml d'eau. Le précipité formé est isolé par filtration, immédiatement après la fin de l'addition. Le solide obtenu est lavé à l'eau froide, puis à l'éther éthylique. Après séchage, il est utilisé dans l'étape suivante sans autre purification. Rdt : 46,2 g (quantitatif), F = 163 - 165°C. Un échantillon analytique a été obtenu par recristallisation dans le méthanol. F = 166 - 167°C.

| Analyse centésimale : C₇H₁₀N₄O₃ (M = 198,18) | | | |
|---|---|---|---|
| | C % | H % | N % |
| calculé | 42,42 | 5,09 | 28,27 |
| trouvé | 42,57 | 5,08 | 28,40 |

IR : ν̅ (C = O) = 1660 cm⁻¹
R.M.N. (DMSO d₆) : δ = 1,15 (3H,t) ; 3,55 (2H,q) ; 4,25 (2H,s) ; 9,15 (1H,pic échangeable avec CF₃COOD) ; 11,15 (1H,pic échangeable avec CF₃COOD) ; 12,15 (1H,pic échangeable avec CF₃COOD).

### c) 2-(Ethoxyméthyl)ptéridin-4(3H)-one

Une solution de 19,1 g (0,11 mole) de dithionite de sodium dans 88 ml d'eau est ajoutée goutte à goutte, à une suspension maintenue à 20°C, de 10,0 g (0,050 mole) de 6-amino-2-(éthoxyméthyl)-5-nitrosopyrimidin-4(3H)-one dans 50 ml d'eau. Après agitation pendant 30 minutes à température ambiante, le milieu réactionnel, toujours maintenu à 20°C, est additionné de 47,9 g (0,33 mole) d'une solution aqueuse à 40 % de glyoxal. L'agitation est poursuivie pendant 20 heures à température ambiante. La solution obtenue est alors extraite au chlorure de méthylène. Ces extraits organiques sont séchés sur sulfate de sodium et concentrés à sec sous pression réduite. Le résidu solide est recristallisé dans le méthanol en présente de Norit. Rdt : 6,5 g (63 %), F = 168 - 169°C. Le produit est identique à celui obtenu dans l'exemple 1.

### Exemple 26 :

### 2-(Ethoxyméthyl)ptéridin-4(3H)-one

### a) 5,6-Diamino-2-(éthoxyméthyl)pyrimidin-4(3H)-one

Un mélange de 12,4 g (0,063 mole) de 6-amino-2-(éthoxyméthyl)-5-nitrosopyrimidin-4(3H)-one brute, non lavée à l'eau, et de 1 g de nickel de Raney dans 430 ml de méthanol est introduit dans un autoclave. La pression initiale d'hydrogène est fixée à 75 bars ; l'autoclave est agité pendant 2 heures à température ambiante. Après dégazage, la partie insoluble est isolée par filtration et lavée au N,N-diméthylformamide. Le filtrat et la solution de lavage rassemblés sont concentrés à sec sous pression réduite. Le résidu est lavé à l'éther éthylique et recristallisé dans un mélange d'éthanol et d'eau. Rdt : 5,0 g (43 %), F = 173 - 175°C.
R.M.N. (DMSO d₆) : δ = 1,1 (3H,t) ; 3,5 (2H,q) ; 4,1 (2H,s) ; 5,9 (5H,pic échangeable avec CF₃COOD).

### b) Hémimaléate de 5,6-diamino-2-(éthoxyméthyl)pyrimidin-4(3H)-one

Un mélange de 3,4 g (0,0185 mole) de 5,6-diamino-2-(éthoxyméthyl)pyrimidin-4(3H)-one, de 4,4 g (0,0379 mole) d'acide maléique, de 300 ml d'éthanol absolu et de 100 ml de méthanol est porté à reflux. La faible partie insoluble est éliminée par filtration à chaud. Après refroidissement, le précipité formé est isolé par Filtration ; il est lavé à l'éther éthylique et recristallisé dans l'éthanol absolu. Rdt : 2,0 g (45 %), F = 192 - 194°C.

| Analyse centésimale : C₇H₁₂N₄O₂, 1/2 (C₄H₄0₄) (M = 242,235) | | | |
|---|---|---|---|
| | C % | H % | N % |
| calculé | 44,63 | 5,83 | 23,13 |
| trouvé | 44,62 | 5,73 | 23,47 |

R.M.N. (DMSO d₆ + CF₃COOD) : δ = 1,1 (3H,t) ; 3,45 (2H,q) ; 4,15 (2H,s) ; 6,15 (1H,s).

### c) 2-(Ethoxyméthyl)ptéridin-4(3H)-one

On ajoute 3,5 g (0,024 mole) d'une solution aqueuse à 40 % de glyoxal, à une suspension de 3,7 g (0,020 mole) de 5,6-diamino-2-(éthoxyméthyl)pyrimidin-4(3H)-one dans 30 ml d'eau. Le mélange est porté progressivement à reflux et maintenu ainsi pendant 1 heure. Après addition de Norit, le reflux est poursuivi pendant encore 10 minutes, puis le milieu réactionnel est filtré. La solution aqueuse obtenue est extraite au chlorure de méthylène. Ces extraits organiques sont traités comme dans le paragraphe c de l'exemple 25. Rdt : 2,5 (61 %), F = 168 - 169°C. Le produit est identique à celui obtenu dans l'exemple 1.

## Revendications (Revendications pour l'(les) Etat(s) contractant(s) suivant(s): AT, BE, CH, DE, DK, FR, GB, IT, LI, LU, NL, SE)

1. Ptéridin-4(3H)-ones caractérisées par la formule 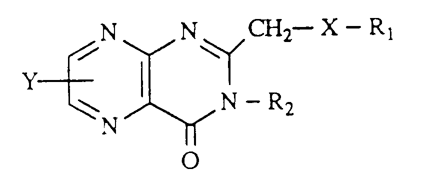 dans laquelle X est un atome d'oxygène ou un atome de soufre, Y est un atome d'hydrogène, un radical (C₁-C₄)-alcoyle en particulier un radical méthyle en position 6 ou un groupe hydroxyle en position 7, R, est un atome d'hydrogène, un radical (C₁-C₄)-alcoyle, un radical phényle éventuellement substitué par un à trois groupes choisis parmi (C₁-C₄)-alcoyle, (C₁-C₄)-alcoxy, halogène, hydroxyle et acétyle, un radical benzyle, le groupe méthoxyméthyle,
le groupe acétyle, le groupe 2-acétoxyéthyle ou le groupe 2,2,2-trifluoroéthyle et R₂ est un atome d'hydrogène ou un radical (C₁-C₄)-alcoyle en particulier un radical méthyle; et leurs sels de métaux alcalins pharmaceutiquement acceptables.

2. Ptéridin-4(3H)-ones selon la revendication 1, caractérisées en ce que X est un atome d'oxygène, Y est un atome d'hydrogène ou un groupe hydroxyle en position 7 et R₂ est un atome d'hydrogène; et leur sels de métaux alcalins pharmaceutiquement acceptables.

3. Ptéridin-4(3H)-ones selon la revendication 1, caractérisées en ce que R₁ est un radical (C₁-C₄)-alcoyle, le radical phényle ou le radical benzyle; et leur sels de métaux alcalins pharmaceutiquement acceptables.

4. Ptéridin-4(3H)-one selon la revendication 1, la 2-(méthoxyméthyl)ptéridin-4(3H)-one, et ses sels de métaux alcalins pharmaceutiquement acceptables.

5. Ptéridin-4(3H)-one selon la revendication 1, la 2-(éthoxyméthyl)ptéridin-4(3H)-one, et ses sels de métaux alcalins pharmaceutiquement acceptables.

6. Ptéridin-4(3H)-one selon la revendication 1, la 2-(propoxyméthyl)ptéridin-4(3H)-one, et ses sels de métaux alcalins pharmaceutiquement acceptables.

7. Ptéridin-4(3H)-one selon la revendication 1, la 2-(phénoxyméthyl)ptéridin-4(3H)-one, et ses sels de métaux alcalins pharmaceutiquement acceptables.

8. Ptéridin-4(3H)-one selon la revendication 1, la 2-(benzyloxyméthyl)ptéridin-4(3H)-one, et ses sels de métaux alcalins pharmaceutiquement acceptables.

9. Ptéridin-4(3H)-one selon la revendication 1, la 2-(éthoxyméthyl)-7-hydroxyptéridin-4(3H)-one, et ses sels de métaux alcalins pharmaceutiquement acceptables.

10. Procédé de préparation des composés selon la revendication 1, caractérisé en ce qu'on traite un 3-aminopyrazine-2-carboxamide de formule 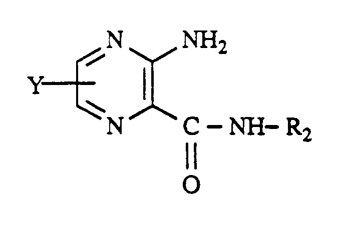 par un orthoester de formule R₁XCH₂C(OR₃)₃, X, Y, R₁ et R₂ ayant les significations données dans la revendication 1, R₃ étant un radical alcoyle.

11. Procédé de préparation des composés selon la revendication 1 pour lesquels R₂ est un atome d'hydrogène, caractérisé en ce qu'on hydrolyse dans l'eau alcaline une 4-aminoptéridine de formule 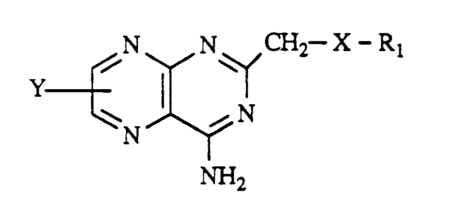 X, Y et R₁ ayant les significations données dans la revendication 1.

12. Procédé de préparation des composés selon la revendication 1 pour lesquels Y et R₂ sont des atomes d'hydrogène, caractérisé en ce qu'on traite une 5,6-diaminopyrimidin-4(3H)-one de formule 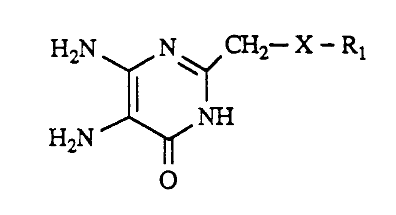 par le glyoxal, X et R₁ ayant les significations données dans la revendication 1.

13. Produits intermédiaires dans la préparation des composés selon la revendication 11 caractérisés par la formule 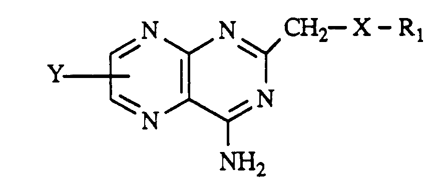 X, Y et R₁ ayant les significations données dans la revendication 1.

14. Produits intermédiaires dans la préparation des composés selon la revendication 12 caractérisés par la formule 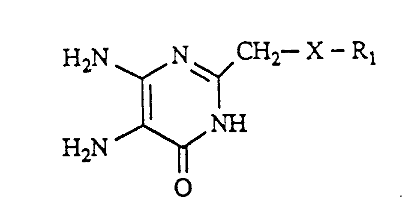 X et R₁ ayant les significations données dans la revendication 1.

15. Composition pharmaceutique caractérisée en ce qu'elle comporte au moins une ptéridin-4(3H)-one telle que définie à la revendication 1 et un excipient pharmaceutiquement acceptable.

16. Composition pharmaceutique selon la revendication 15, caractérisée en ce qu'elle est sous forme d'aérosols, comprimés, comprimés dragéifiés, gélules, soluté injectable, pommade, poudre, solution ou suppositoires.

17. Utilisation des ptéridin-4(3H)-ones telles que définies à la revendication 1 pour la préparation de médicaments utiles dans le traitement des allergies.

## Revendications (Revendications pour l'(les) Etat(s) contractant(s) suivant(s): ES)

1. Procédé de préparation des ptéridin-4(3H)-ones de formule 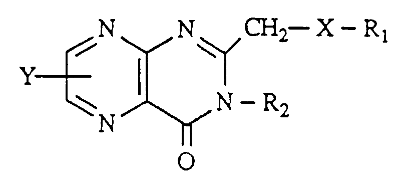 dans laquelle X est un atome d'oxygène ou un atome de soufre, Y est un atome d'hydrogène, un radical (C₁-C₄)-alcoyle en particulier un radical méthyle en position 6 ou un groupe hydroxyle en position 7, R₁ est un atome d'hydrogène, un radical (C₁-C₄)-alcoyle, un radical phényle éventuellement substitué par un à trois groupes choisis parmi (C₁-C₄)-alcoyle, (C₁-C₄)-alcoxy, halogène, hydroxyle et acétyle, un radical benzyle, le groupe méthoxyméthyle,
le groupe acétyle, le groupe 2-acétoxyéthyle ou le groupe 2,2,2-trifluoroéthyle et R₂ est un atome d'hydrogène ou un radical (C₁-C₄)-alcoyle en particulier un radical méthyle; et leurs sels de métaux alcalins pharmaceutiquement acceptables, caractérisé en ce qu'on traite un 3-aminopyrazine-2-carboxamide de formule 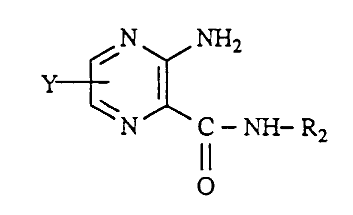 par un orthoester de formule R₁XCH₂C(OR₃)₃, X, Y, R₁ et R₂ ayant les significations données ci-dessus , R₃ étant un radical alcoyle , ou
lorsque R₂ est un atome d'hydrogène, on hydrolyse dans l'eau alcaline une 4-aminoptéridine de formule 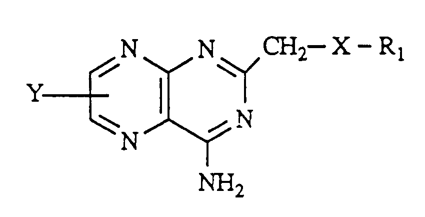 X, Y et R₁ ayant les significations données ci-dessus, ou lorsque Y et R₂ sont des atomes d'hydrogène, on traite une 5,6-diaminopyrimidin-4(3H)-one de formule 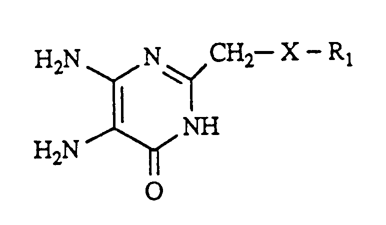 wpar le glyoxal, X et R₁ ayant les significations données ci-dessus.

2. Procédé selon la revendication 1, caractérisé en ce que les ptéridin-4(3H)-ones sont celles où X est un atome d'oxygène, Y est un atome d'hydrogène ou un groupe hydroxyle en position 7 et R₂ est un atome d'hydrogène ; et leurs sels de métaux alcalins pharmaceutiquement acceptables.

3. Procédé selon la revendication 1, caractérisé en ce que les ptéridin-4(3H)-ones sont celles où R₁ est un radical (C₁-C₄) alcoyle, le radical phényle ou le radical benzyle; et leur sels de métaux alcalins pharmaceutiquement acceptables.

4. Procédé selon la revendication 1, caractérisé en ce que la ptéridin-4(3H)-one est la 2-(méthoxyméthyl)ptéridin-4(3H)-one, et ses sels de métaux alcalins pharmaceutiquement acceptables.

5. Procédé selon la revendication 1, caractérisé en ce que la ptéridin-4(3H)-one est la 2-(éthoxyméthyl)ptéridin-4(3H)-one, et ses sels de métaux alcalins pharmaceutiquement acceptables.

6. Procédé selon la revendication 1, caractérisé en ce que la ptéridin-4(3H)-one est la 2-(propoxyméthyl)ptéridin-4(3H)-one, et ses sels de métaux alcalins pharmaceutiquement acceptables.

7. Procédé selon la revendication 1, caractérisé en ce que la ptéridin-4(3H)-one est la 2-(phénoxyméthyl)ptéridin-4(3H)-one, et ses sels de métaux alcalins pharmaceutiquement acceptables.

8. Procédé selon la revendication 1, caractérisé en ce que la ptéridin-4(3H)-one est la 2-(benzyloxyméthyl)ptéridin-4(3H)-one, et ses sels de métaux alcalins pharmaceutiquement acceptables.

9. Procédé selon la revendication 1, caractérisé en ce que la ptéridine-4(3H)-one est la 2-(éthoxyméthyl)-7-hydroxyptéridin-4(3H)-one, et ses sels de métaux alcalins pharmaceutiquement acceptables.

10. Procédé de préparation des composés de formule 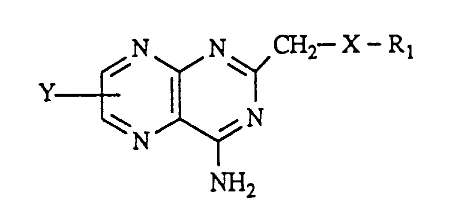 X, Y et R₁ ayant les significations données dans la revendication 1, intermédiaires dans le procédé de préparation de la revendication 1, caractérisé en ce que l'on condense un 3-aminopyrazine-2-carbonitrile de formule 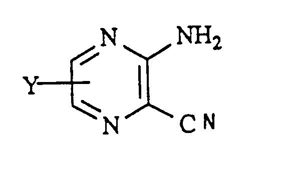 sur une acétamidine de formule 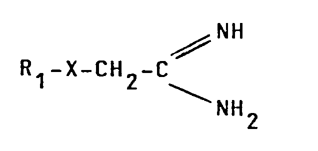 dans lesquelles
X, Y et R₁ ont les significations données précédemment.

11. Procédé de préparation des 5,6-diaminopyrimidin-4(3H)-ones de formule 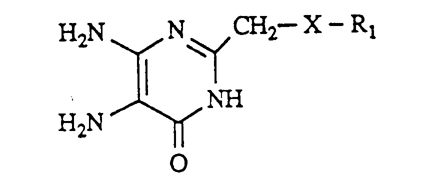 X et R₁ ayant les significations données dans la revendication 1, intermédiaires dans le procédé de préparation de la revendication 1, caractérisé en ce qu'une acétamidine de formule 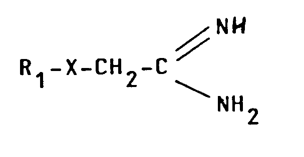 X et R₁ ayant les significations ci-dessus, est condensée sur l'iminoéther du cyanoacétate d'éthyle de formule 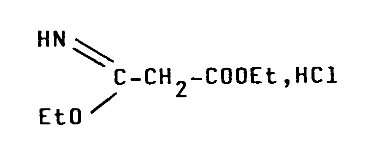 pour donner une 6-aminopyrimidin-4(3H)-one de formule 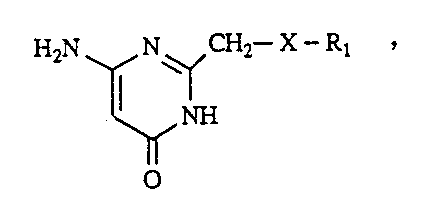 X et R₁ ayant les significations ci-dessus
qui est nitrosé pour donner une 6-amino-5-nitrosopyrimidin-4(3H)-one de formule 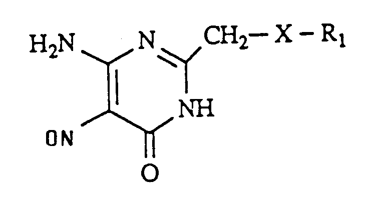 X et R₁ ayant les significations ci-dessus
dont la réduction fournit la 5,6-diaminopyrimidin-4(3H)-one.

12. Procédé de préparation de compositions pharmaceutiques, caractérisé en ce que l'on mélange au moins un composé selon la revendication 1 avec un excipient pharmaceutiquement acceptable.

13. Procédé selon la revendication 12, caractérisé en ce que l'excipient est propre à la préparation de compositions sous forme d'aérosols, comprimés, comprimés dragéifiés, gélules, soluté injectable, pommade, poudre, solution ou suppositoires.

## Revendications (Revendications pour l'(les) Etat(s) contractant(s) suivant(s): GR)

1. Ptéridin-4(3H)-ones caractérisées par la formule 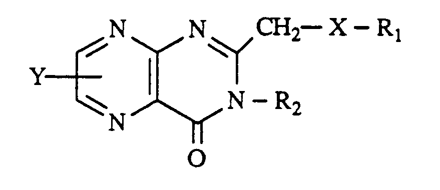 dans laquelle X est un atome d'oxygène ou un atome de soufre, Y est un atome d'hydrogène, un radical (C₁-C₄)-alcoyle en particulier un radical méthyle, en position 6 ou un groupe hydroxyle en position 7, R₁ est un atome d'hydrogène, un radical (C₁-C₄)alcoyle, un radical phényle éventuellement substitué par un à trois groupes choisis parmi (C₁-C₄)-alcoyle, (C₁-C₄)-alcoxy, halogène, hydroxyle et acétyle, un radical benzyle, le groupe méthoxyméthyle,
le groupe acétyle, le groupe 2-acétoxyéthyle ou le groupe 2,2,2-trifluoroéthyle et R₂ est un atome d'hydrogène ou un radical (C₁-C₄)-alcoyle en particulier un radical méthyle; et leurs sels de métaux alcalins pharmaceutiquement acceptables.

2. Ptéridin-4(3H)-ones selon la revendication 1, caractérisées en ce que X est un atome d'oxygène, Y est un atome d'hydrogène ou un groupe hydroxyle en position 7 et R₂ est un atome d'hydrogène; et leur sels de métaux alcalins pharmaceutiquement acceptables.

3. Ptéridin-4(3H)-ones selon la revendication 1, caractérisées en ce que R₁ est un radical (C₁-C₄)-alcoyle, le radical phényle ou le radical benzyle; et leur sels de métaux alcalins pharmaceutiquement acceptables.

4. Ptèridin-4(3H)-one selon la revendication 1, la 2-(méthoxyméthyl)ptéridin-4(3H)-one, et ses sels de métaux alcalins pharmaceutiquement acceptables.

5. Ptéridin-4(3H)-one selon la revendication 1, la 2-(éthoxyméthyl)ptéridin-4(3H)-one, et ses sels de métaux alcalins pharmaceutiquement acceptables.

6. Ptéridin-4(3H)-one selon la revendication 1, la 2-(propoxyméthyl)ptéridin-4(3H)-one, et ses sels de métaux alcalins pharmaceutiquement acceptables.

7. Ptéridin-4(3H)-one selon la revendication 1, la 2-(phénoxyméthyl)ptéridin-4(3H)-one, et ses sels de métaux alcalins pharmaceutiquement acceptables.

8. Ptéridin-4(3H)-one selon la revendication 1, la 2-(benzyloxyméthyl)ptéridin-4(3H)-one, et ses sels de métaux alcalins pharmaceutiquement acceptables.

9. Ptéridin-4(3H)-one selon la revendication 1, la 2-(éthoxyméthyl)-7-hydroxyptéridin-4(3H)-one, et ses sels de métaux alcalins pharmaceutiquement acceptables.

10. Procédé de préparation des composés selon la revendication 1, caractérisé en ce qu'on traite un 3-aminopyrazine-2-carboxamide de formule 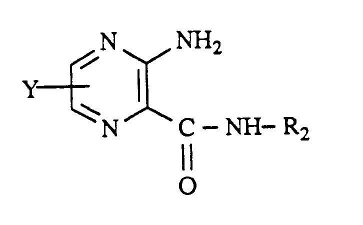 par un orthoester de formule R₁XCH₂C(OR₃)₃, X, Y, R₁ et R₂ ayant les significations données dans la revendication 1, R₃ étant un radical alcoyle.

11. Procédé de préparation des composés selon la revendication 1 pour lesquels R₂ est un atome d'hydrogène, caractérisé en ce qu'on hydrolyse dans l'eau alcaline une 4-aminoptéridine de formule 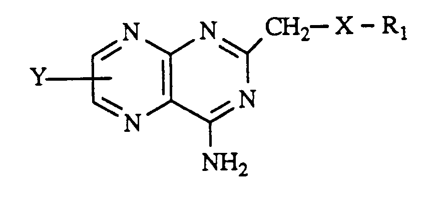 X, Y et R₁ ayant les significations données dans la revendication 1.

12. Procédé de préparation des composés selon la revendication 1 pour lesquels Y et R₂ sont des atomes d'hydrogène, caractérisé en ce qu'on traite une 5,6-diaminopyrimidin-4(3H)-one de formule 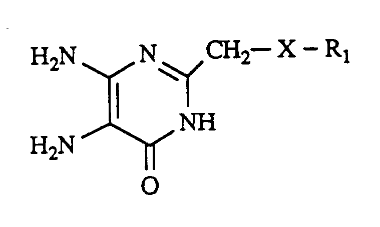 par le glyoxal, X et R₁ ayant les significations données dans la revendication 1.

13. Produits intermédiaires dans la préparation des composés selon la revendication 11 caractérisés par la formule 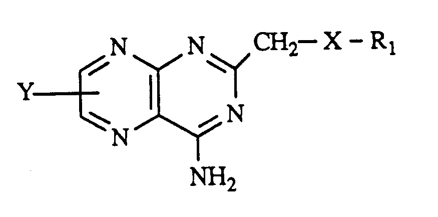 X, Y et R₁ ayant les significations données dans la revendication 1.

14. Produits intermédiaires dans la préparation des composés selon la revendication 12 caractérisés par la formule 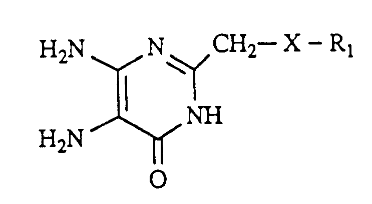 X et R₁ ayant les significations données dans la revendication 1.

15. Procédé de préparation de compositions pharmaceutiques, caractérisé en ce que l'on mélange au moins une ptéridin -4(3H)-one selon la revendication 1, avec un excipient pharmaceutiquement acceptable.

16. Procédé selon la revendication 15, caractérisé en ce quel'excipient est propre à la préparation de compositions sous forme d'aérosols, comprimés, comprimés dragéifiés, gélules, soluté injectable, pommade, poudre, solution ou suppositoires.

## Claims (Claims for the following Contracting State(s): AT, BE, CH, DE, DK, FR, GB, IT, LI, LU, NL, SE)

1. Pteridin-4(3H)-ones characterised by the formula 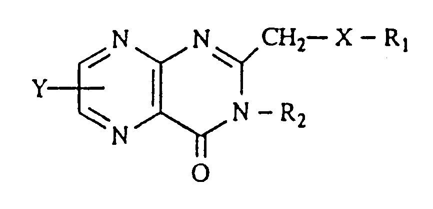 wherein X is an oxygen or sulphur atom, Y is a hydrogen atom, a (C₁₋₄)alkyl radical, particularly a methyl radical in position 6 or a hydroxy group in position 7, R₁ is a hydrogen atom, a (C₁₋₄)alkyl radical, a phenyl radical optionally substituted by one to three groups selected from (C₁₋₄)alkyl, (C₁₋₄)alkoxy, halogen, hydroxy or acetyl, a benzyl radical, a methoxymethyl group,
the acetyl group, the 2-acetoxyethyl group or the group 2,2,2-trifluoroethyl, and R₂ is a hydrogen atom or a (C₁₋₄)alkyl radical, particularly a methyl radical; and the pharmaceutically acceptable alkali metal salts thereof.

2. Pteridin-4(3H)-ones according to claim 1, characterised in that X is an oxygen atom, Y is a hydrogen atom or a hydroxy group in position 7 and R₂ is a hydrogen atom; and the pharmaceutically acceptable alkali metal salts thereof.

3. Pteridin-4(3H)-ones according to claim 1, characterised in that R₁ is a (C₁₋₄)alkyl radical, a phenyl radical or a benzyl radical; and the pharmaceutically acceptable alkali metal salts thereof.

4. Pteridin-4(3H)-one according to claim 1, 2-(methoxymethyl)pteridin-4(3H)-one and the pharmaceutically acceptable alkali metal salts thereof.

5. Pteridin-4(3H)-one according to claim 1, 2-(ethoxymethyl)pteridin-4(3H)-one and the pharmaceutically acceptable alkali metal salts thereof.

6. Pteridin-4(3H)-one according to claim 1, 2-(propoxymethyl)pteridin-4(3H)-one and the pharmaceutically acceptable alkali metal salts thereof.

7. Pteridin-4(3H)-one according to claim 1, 2-(phenoxymethyl)pteridin-4(3H)-one and the pharmaceutically acceptable alkali metal salts thereof.

8. Pteridin-4(3H)-one according to claim 1, 2-(benzyloxymethyl)pteridin-4(3H)-one and the pharmaceutically acceptable alkali metal salts thereof.

9. Pteridin-4(3H)-one according to claim 1, 2-(ethoxymethyl)-7-hydroxypteridin-4(3H)-one and the pharmaceutically acceptable alkali metal salts thereof.

10. Process for preparing the compounds according to claim 1, characterised in that a 3-aminopyrazine-2-carboxamide of the formula 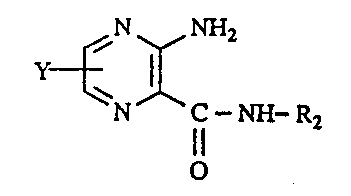 is treated with an orthoester of formula R₁XCH₂C(OR₃)₃, wherein X, Y, R₁ and R₂ have the meanings given in claim 1 and R₃ is an alkyl radical.

11. Process for preparing the compounds according to claim 1 wherein R₂ is a hydrogen atom, characterised in that a 4-aminopteridine of formula 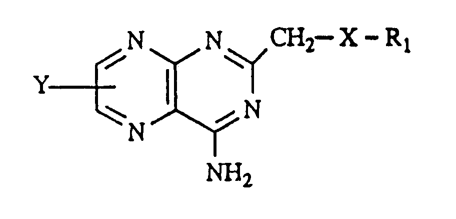 wherein X, Y and R₁ have the meanings given in claim 1, is hydrolysed in alkaline water.

12. Process for preparing compounds according to claim 1 wherein Y and R₂ are hydrogen atoms, characterised in that a 5,6-diaminopyrimidin-4(3H)-one of the formula 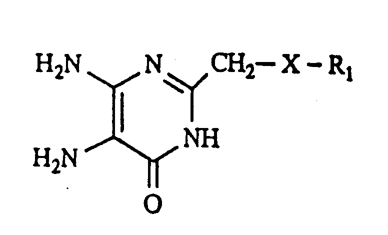 wherein X and X₁ have the meanings given in claim 1, is treated with glyoxal.

13. Intermediate products in the preparation of the compounds according to claim 11, characterised by the formula 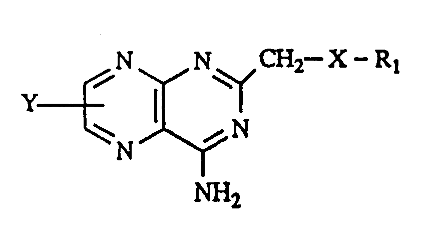 wherein X, Y and R₁ have the meanings given in claim 1.

14. Intermediate products in the preparation of the compounds according to claim 12, characterised by the formula 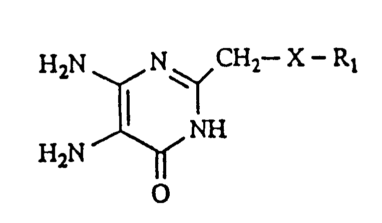 wherein X and R₁ have the meanings given in claim 1.

15. Pharmaceutical composition, characterised in that it contains at least one pteridin-4(3H)-one as defined in claim 1 and a pharmaceutically acceptable excipient.

16. Pharmaceutical composition according to claim 15, characterised in that it is in the form of an aerosol, tablets, coated tablets, gelatin capsules, injectable solution, ointment, powder, solution or suppositories.

17. Use of the pteridin-4(3H)-ones as defined in claim 1 for preparing drugs which can be used in the treatment of allergies.

## Claims (Claims for the following Contracting State(s): ES)

1. Process for preparing pteridin-4(3H)-ones of the formula 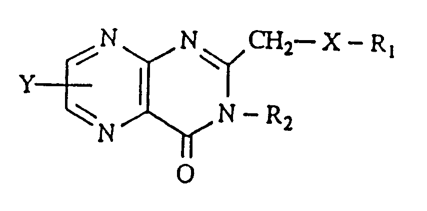 wherein X is an oxygen or sulphur atom, Y is a hydrogen atom, a (C₁₋₄)alkyl radical, particularly a methyl radical in position 6 or a hydroxy group in position 7, R₁ is a hydrogen atom, a (C₁₋₄)alkyl radical, a phenyl radical optionally substituted by one to three groups selected from (C₁₋₄)alkyl, (C₁₋₄)alkoxy, halogen, hydroxy or acetyl, a benzyl radical, a methoxymethyl group,
the acetyl group, the 2-acetoxyethyl group or the group 2,2,2-trifluoroethyl, and R₂ is a hydrogen atom or a (C₁₋₄)alkyl radical, particularly a methyl radical; and the pharmaceutically acceptable alkali metal salts thereof, characterised in that a 3-aminopyrazine-2-carboxamide of the formula 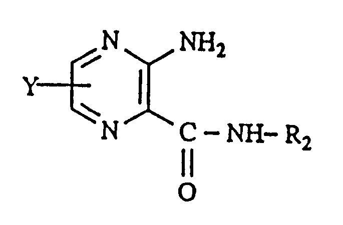 is treated with an orthoester of formula R₁XCH₂C(OR₃)₃, wherein X, Y, R₁ and R₂ have the meanings given hereinbefore and R₃ is an alkyl radical, or
when R₂ is a hydrogen atom, a 4-aminopteridine of formula 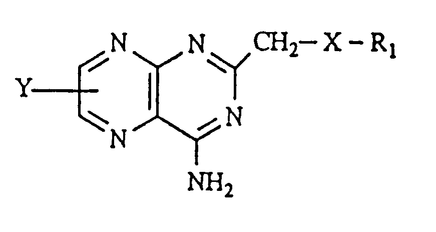 wherein X, Y and R₁ are as hereinbefore defined, is hydrolysed in alkaline water, or
when Y and R₂ are hydrogen atoms, a 5,6-diaminopyrimidin-4(3H)-one of the formula 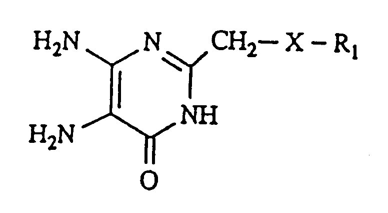 wherein X and R₁ are as hereinbefore defined, is treated with glyoxal.

2. Process according to claim 1, characterised in that the pteridin-4(3H)-ones are those wherein X is an oxygen atom, Y is a hydrogen atom or a hydroxy group in the 7-position and R₂ is a hydrogen atom; and the pharmaceutically acceptable alkali metal salts thereof.

3. Process according to claim 1, characterised in that the pteridin-4(3H)-ones are those wherein R₁ is a (C₁₋₄)alkyl radical, a phenyl radical or a benzyl radical; and the pharmaceutically acceptable alkali metal salts thereof.

4. Process according to claim 1, characterised in that the pteridin-4(3H)-one is 2-(methoxymethyl)pteridin-4(3H)-one, and the pharmaceutically acceptable alkali metal salts thereof.

5. Process according to claim 1, characterised in that the pteridin-4(3H)-one is 2-(ethoxymethyl)pteridin-4(3H)-one, and the pharmaceutically acceptable alkali metal salts thereof.

6. Process according to claim 1, characterised in that the pteridin-4(3H)-one is 2-(propoxymethyl)pteridin-4(3H)-one, and the pharmaceutically acceptable alkali metal salts thereof.

7. Process according to claim 1, characterised in that the pteridin-4(3H)-one is 2-(phenoxymethyl)pteridin-4(3H)-one, and the pharmaceutically acceptable alkali metal salts thereof.

8. Process according to claim 1, characterised in that the pteridin-4(3H)-one is 2-(benzyloxymethyl)pteridin-4(3H)-one, and the pharmaceutically acceptable alkali metal salts thereof.

9. Process according to claim 1, characterised in that the pteridin-4(3H)-one is 2-(ethoxymethyl)-7-hydroxypteridin-4(3H)-one, and the pharmaceutically acceptable alkali metal salts thereof.

10. Process for preparing compounds of formula 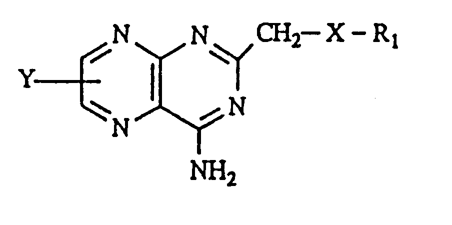 wherein X, Y and R₁ have the meanings given in claim 1, which are intermediate in the preparation process of claim 1, characterised in that a 3-aminopyrazine-2-carbonitrile of formula 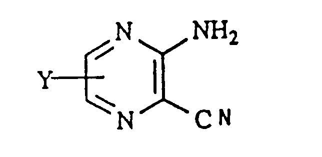 is condensed with an acetamidine of formula 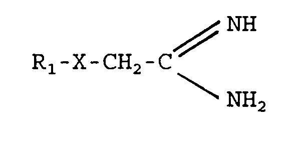 wherein
X, Y and R₁ are as hereinbefore defined.

11. Process for preparing the 5,6-diaminopyrimidin-4(3H)-ones of the formula 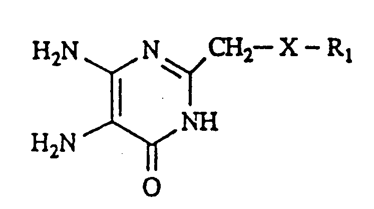 wherein X and R₁ are defined as in claim 1, which are intermediate in the preparation process of claim 1, characterised in that an acetamidine of formula 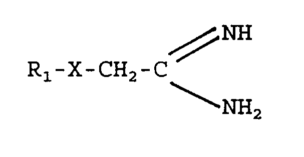 wherein X and R₁ are as hereinbefore defined is condensed with the iminoether of ethyl cyanoacetate of the formula 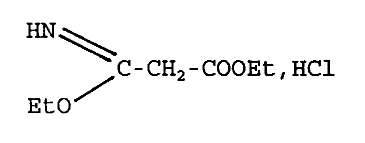 to give a 6-aminopyrimidin-4(3H)-one of formula 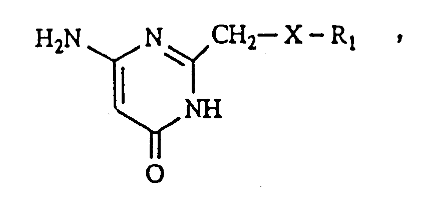 wherein X and R₁ are defined as hereinbefore, which is nitrosed to give a 6-amino-5-nitrosopyrimidin-4(3H)-one of the formula 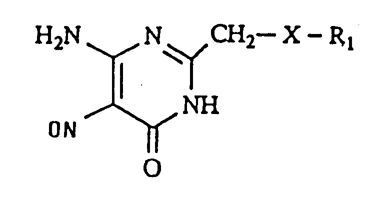 wherein X and R₁ are as hereinbefore defined, reduction of which yields 5,6-diaminopyrimidin-4(3H)-one.

12. Process for preparing pharmaceutical compositions, characterised in that at least one compound according to claim 1 is mixed with a pharmaceutically acceptable excipient.

13. Process according to claim 12, characterised in that the excipient is suitable for the preparation of compositions in the form of aerosols, tablets, coated tablets, gelatin capsules, injectable solutions, ointments, powders, solutions or suppositories.

## Claims (Claims for the following Contracting State(s): GR)

1. Pteridin-4(3H)-ones characterised by the formula 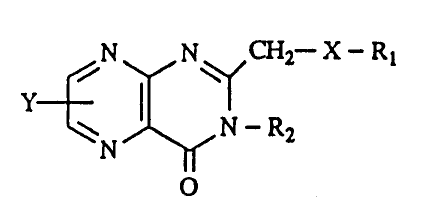 wherein X is an oxygen or sulphur atom, Y is a hydrogen atom, a (C₁₋₄)alkyl radical, particularly a methyl radical in position 6 or a hydroxy group in position 7, R₁ is a hydrogen atom, a (C₁₋₄)alkyl radical, a phenyl radical optionally substituted by one to three groups selected from (C₁₋₄)alkyl, (C₁₋₄)alkoxy, halogen, hydroxy or acetyl, a benzyl radical, a methoxymethyl group,
the acetyl group, the 2-acetoxyethyl group or the group 2,2,2-trifluoroethyl, and R₂ is a hydrogen atom or a (C₁₋₄)alkyl radical, particularly a methyl radical; and the pharmaceutically acceptable alkali metal salts thereof.

2. Pteridin-4(3H)-ones according to claim 1, characterised in that X is an oxygen atom, Y is a hydrogen atom or a hydroxy group in position 7 and R₂ is a hydrogen atom; and the pharmaceutically acceptable alkali metal salts thereof.

3. Pteridin-4(3H)-ones according to claim 1, characterised in that R₁ is a (C₁₋₄)alkyl radical, a phenyl radical or a benzyl radical; and the pharmaceutically acceptable alkali metal salts thereof.

4. Pteridin-4(3H)-one according to claim 1, 2-(methoxymethyl)pteridin-4(3H)-one and the pharmaceutically acceptable alkali metal salts thereof.

5. Pteridin-4(3H)-one according to claim 1, 2-(ethoxymethyl)pteridin-4(3H)-one and the pharmaceutically acceptable alkali metal salts thereof.

6. Pteridin-4(3H)-one according to claim 1, 2-(propoxymethyl)pteridin-4(3H)-one and the pharmaceutically acceptable alkali metal salts thereof.

7. Pteridin-4(3H)-one according to claim 1, 2-(phenoxymethyl)pteridin-4(3H)-one and the pharmaceutically acceptable alkali metal salts thereof.

8. Pteridin-4(3H)-one according to claim 1, 2-(benzyloxymethyl)pteridin-4(3H)-one and the pharmaceutically acceptable alkali metal salts thereof.

9. Pteridin-4(3H)-one according to claim 1, 2-(ethoxymethyl)-7-hydroxypteridin-4(3H)-one and the pharmaceutically acceptable alkali metal salts thereof.

10. Process for preparing the compounds according to claim 1, characterised in that a 3-aminopyrazine-2-carboxamide of the formula 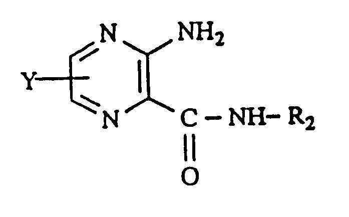 is treated with an orthoester of formula R₁XCH₂C(OR₃)₃, wherein X, Y, R₁ and R₂ have the meanings given in claim 1 and R₃ is an alkyl radical.

11. Process for preparing the compounds according to claim 1 wherein R₂ is a hydrogen atom, characterised in that a 4-aminopteridine of formula 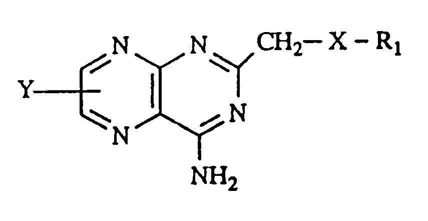 wherein X, Y and R₁ have the meanings given in claim 1, is hydrolysed in alkaline water.

12. Process for preparing compounds according to claim 1 wherein Y and R₂ are hydrogen atoms, characterised in that a 5,6-diaminopyrimidin-4(3H)-one of the formula 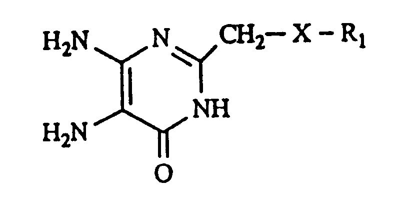 wherein X and X₁ have the meanings given in claim 1, is treated with glyoxal.

13. Intermediate products in the preparation of the compounds according to claim 11, characterised by the formula 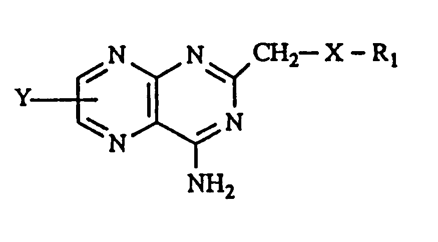 wherein X, Y and R₁ have the meanings given in claim 1.

14. Intermediate products in the preparation of the compounds according to claim 12, characterised by the formula 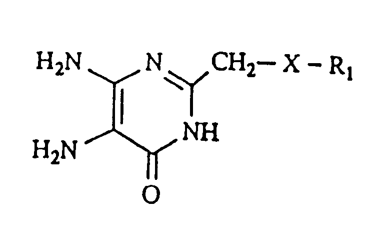 wherein X and R₁ have the meanings given in claim 1.

15. Process for preparing pharmaceutical compositions, characterised in that at least one pteridin-4(3H)-one according to claim 1 is mixed with a pharmaceutically acceptable excipient.

16. Process according to claim 15, characterised in that the excipient is suitable for the preparation of compositions in the form of aerosols, tablets, coated tablets, gelatin capsules, injectable solutions, ointments, powders, solutions or suppositories.

## Patentansprüche (Patentansprüche für folgende(n) Vertragsstaat(en): AT, BE, CH, DE, DK, FR, GB, IT, LI, LU, NL, SE)

1. Pteridin-4(3H)-one der Formel 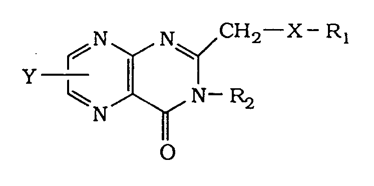 in der X ein Sauerstoffatom oder ein Schwefelatom,
Y ein Wasserstoffatom, eine C₁-C₄-Alkylgruppe, insbesondere eine Methylgruppe, in der 6-Stellung oder eine Hydroxylgruppe in der 7-Stellung,
R₁ ein Wasserstoffatom, eine C₁-C₄-Alkylgruppe. eine Phenylgruppe, die gegebenenfalls durch eine bis drei Gruppen ausgewählt aus C₁-C₄-Alkylgruppen, C₁-C₄-Alkoxygruppen. Halogenatomen, Hydroxylgruppen und Acetylgruppen substituiert ist, eine Benzylgruppe, die Methoxymethylgruppe, die Acetylgruppe, die 2-Acetoxyethylgruppe oder die 2,2,2-Trifluorethylgruppe und
R₂ ein Wasserstoffatom oder eine C₁-C₄-Alkylgruppe, insbesondere eine Methylgruppe, bedeuten;
und deren pharmazeutisch annehmbare Alkalimetallsalze.

2. Pteridin-4(3H)-one nach Anspruch 1, **dadurch gekennzeichnet**, daß X ein Sauerstoffatom, Y ein Wasserstoffatom oder eine Hydroxylgruppe In der 7-Stellung und R₂ ein Wasserstoffatom bedeuten: und deren pharmazeutisch annehmbare Alkalimetallsalze.

3. Pteridin-4(3H)-one nach Anspruch 1, **dadurch gekennzeichnet**, daß R₁ eine C₁-C₄-Alkylgruppe, die Phenylgruppe oder die Benzylgruppe bedeutet: und deren pharmazeutisch annehmbare Alkalimetallsalze.

4. Pteridin-4(3H)-on nach Anspruch 1, nämlich 2-(Methoxymethyl)-pteridin-4(3H)-on, und dessen pharmazeutisch annehmbare Alkalimetallsalze.

5. Pteridin-4(3H)-on nach Anspruch 1, nämlich 2-(Ethoxymethyl)-pteridin-4(3H)-on, und dessen pharmazeutisch annehmbare Alkalimetallsalze.

6. Pteridin-4(3H)-on nach Anspruch 1, nämlich 2-(Propoxymethyl)-pteridin-4(3H)-on, und dessen pharmazeutisch annehmbare Alkalimetallsalze,

7. Pteridin-4(3H)-on nach Anspruch 1, nämlich 2-(Phenoxymethyl)-pteridin4(3H)-on, und dessen pharmazeutisch annehmbare Alkalimetallsalze.

8. Pteridin-4(3H)-on nach Anspruch 1, nämlich 2-(Benzyloxymethyl)-pteridin-4(3H)-on, und dessen pharmazeutisch annehmbare Alkalimetallsalze.

9. Pteridin-4(3H) -on nach Anspruch 1, nämlich 2-(Ethoxymethyl)-7-hydroxypteridin-4(3H)-on, und dessen pharmazeutisch annehmbare Alkalimetallsalze.

10. Verfahren zur Herstellung der Verbindungen nach Anspruch 1, **dadurch gekennzeichnet**, daß man ein 3-Aminopyrazin-2-carboxamid der Formel 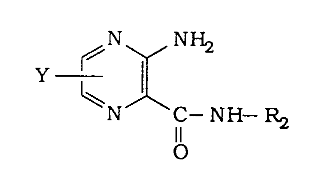 mit einem Orthoester der Formel R₁XCH₂C(OR₃)₃ umsetzt, worin X, Y, R₁ und R₂ die in Anspruch 1 angegebenen Bedeutungen besitzen und R₃ eine Alkylgruppe darstellt.

11. Verfahren zur Herstellung der Verbindungen nach Anspruch 1, worin R₂ ein Wasserstoffatom bedeutet, **dadurch gekennzeichnet**, daß man ein 4-Aminpteridin der Formel 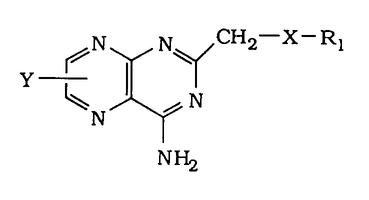 worin X, Y und R₁ die in Anspruch 1 angegebenen Bedeutungen besitzen, in alkalischem Wasser hydrolysiert.

12. Verfahren zur Herstellung der Verbindungen nach Anspruch 1, worin Y und R₂ Wasserstoffatome darstellen**, dadurch gekennzeichnet**, daß man ein 5,6-Diaminopyrimidin-4(3H)-on der Formel 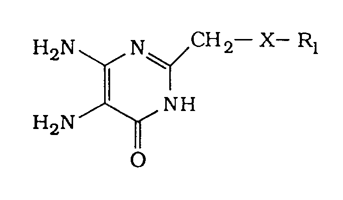 In der X und R₁ die in Anspruch 1 angegebenen Bedeutungen besitzen, mit Glyoxal behandelt.

13. Zwischenprodukte für die Herstellung der Verbindunge nach Anspruch 11, **gekennzeichnet durch** die Formel 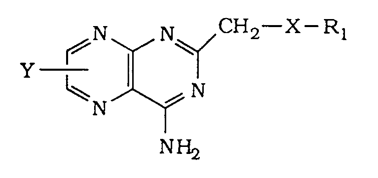 in der X, Y und R₁ die in Anspruch 1 angegebenen Bedeutungen besitzen.

14. Zwischenprodukte für die Herstellung der Verbindungen nach Anspruch 12, **gekennzeichnet durch** die Formel 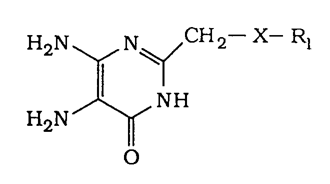 worin X und R₁ die in Anspruch 1 angegebenen Bedeutungen besitzen.

15. Pharmazeutische Zubereitung, **dadurch gekennzeichnet,** daß sie mindestens ein Pteridin-4(3H)-on, wie es in Anspruch 1 definiert ist, und ein pharmazeutisch annehmbares Trägermaterial umfaßt.

16. Pharmazeutische Zubereitung nach Anspruch 15, **dadurch gekennzeichnet**, daß sie in Form eines Aerosols, von Tabletten, dragierten Tabletten, Gelkapseln, injizierbaren Lösungen, Salben, Puder, Lösungen oder Suppositorien vorliegt.

17. Verwendung der Pteridin-4(3H)-one, wie sie in Anspruch 1 definiert sind, für die Herstellung von Arzneimitteln zur Behandlung von Allergien.

## Patentansprüche (Patentansprüche für folgende(n) Vertragsstaat(en): ES)

1. Verfahren zur Hers 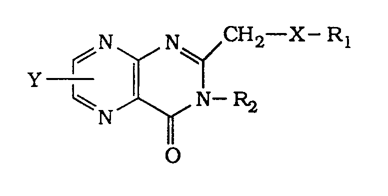 tellung der Pteridin-4(3H)-one der Formel In der X ein Sauerstoffatom oder ein Schwefelatom,
Y ein Wasserstoffatom, eine C₁-C₄-Alkylgruppe, insbesondere eine Methylgruppe, in der 6-Stellung oder eine Hydroxylgruppe in der 7-Stellung.
R₁ ein Wasserstoffatom, eine C₁-C₄-Alkylgruppe, eine Phenylgruppe, die gegebenenfalls durch eine bis drei Gruppen ausgewählt aus C₁-C₄-Alkylgruppen, C₁-C₄-Alkoxygruppen, Halogenatomen, Hydrorylgruppen und Acetylgruppen substituiert ist, eine Benzylgruppe, die Methoxymethylgruppe, die Acetylgruppe, die 2-Acetoxyethylgruppe oder die 2,2,2-Trifluorethylgruppe und
R₂ ein Wasserstoffatom oder eine C₁-C₄-Alkylgruppe, insbesondere eine Methylgruppe, bedeuten;
und von deren pharmazeutisch annehmbaren Alkalimetallsalzen. **dadurch gekennzeichnet**, daß man ein 3-Aminopyrazin-2-carboxamid der Formel 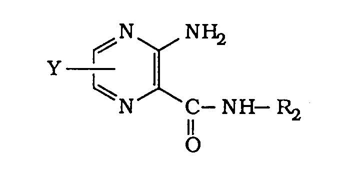 mit einem Orthoester der Formel R₁XCH₂C(OR₃)₃, worin X, Y, R₁ und R₂ die oben angegebenen Bedeutungen besitzen und R₃ eine Alkylgruppe darstellt, behandelt, oder
wenn R₂ ein Wasserstoffatom darstellt, ein 4-Aminopteridin der Formel 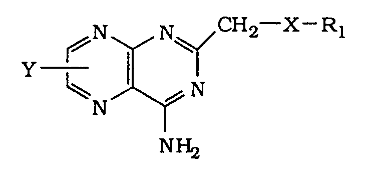 worin X, Y und R₁ die oben angegebenen Bedeutungen besitzen, in alkalischem Wasser hydrolysiert, oder
wenn Y und R₂ Wasserstoffatome darstellen, ein 5,6-Diaminopyrimidin-4(3H)-on der Formel 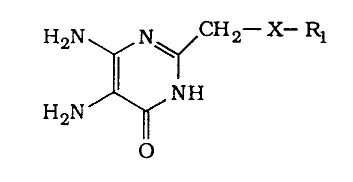 in der X und R₁ die oben angegebenen Bedeutungen besitzen, mit Glyoxal behandelt.

2. Verfahren nach Anspruch 1, **dadurch gekennzeichnet,** daß man Pteridin-4(3H)-one herstellt, worin X ein Sauerstoffatom, Y ein Wasserstoffatom oder eine Hydroxylgruppe in der 7-Stellung und R₂ ein Wasserstoffatom bedeuten, und deren pharmazeutisch annehmbare Alkalimetallsalze.

3. Verfahren nach Anspruch 1, **dadurch gekennzeichnet**, daß man Pteridin-4(3H)-one, worin R₁ eine C₁-C₄-Alkylgruppe, die Phenylgruppe oder die Benzylgruppe darstellt, und deren pharmazeutisch annehmbare Alkalimetallsalze herstellt.

4. Verfahren nach Anspruch 1, **dadurch gekennzeichnet**, daß man als Pteridin-4(3H)-on 2-(Methoxymethyl)-pteridin-4(3H)-on und dessen pharmazeutisch annehmbare Alkalimetallsalze herstellt.

5. Verfahren nach Anspruch 1, **dadurch gekennzeichnet**, daß man als Pteridin-4(3H)-on 2-(Ethoxymethyl)-pteridin-4(3H)-on und dessen pharmazeutisch annehmbare Alkalimetallsalze herstellt.

6. Verfahren nach Anspruch 1, **dadurch gekennzeichnet**, daß man als Pteridin-4(3H)-on 2-(Propoxymethyl)-pteridin-4(3H)-on und dessen pharmazeutisch annehmbare Alkalimetallsalze herstellt.

7. Verfahren nach Anspruch 1**, dadurch gekennzeichnet**, daß man als Pteridin-4(3H)-on 2-(Phenoxymethyl)-pteridin-4(3H)-on und dessen pharmazeutisch annehmbare Alkalimetallsalze herstellt.

8. Verfahren nach Anspruch 1, **dadurch gekennzeichnet**, daß man als Pteridin-4(3H)-on 2-(Benzyloxymethyl)-pteridin-4(3H)-on und dessen pharmazeutisch annehmbare Alkalimetallsalze herstellt.

9. Verfahren nach Anspruch 1, **dadurch gekennzeichnet**, daß man als Pteridin-4(3H)-on 2-(Ethoxymethyl)-7-hydroxypteridin-4(3H)-on und dessen pharmazeutisch annehmbare Alkalimetallsalze herstellt.

10. Verfahren zur Herstellung der Verbindungen der Formel 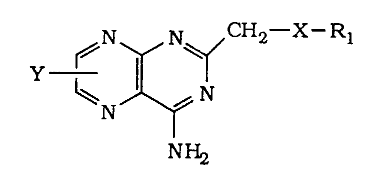 worin X, Y und R₁ die in Anspruch 1 angegebenen Bedeutungen besitzen, und die Zwischenprodukte für das Verfahren nach Anspruch 1 darstellen, **dadurch gekennzeichnet**, daß man ein 3-Aminopyrazin-2-carbonitril der Formel 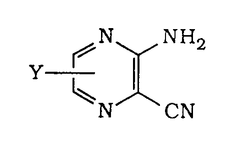 mit einem Acetamidin der Formel 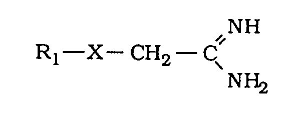 worin X, Y und R₁ die oben angegebenen Bedeutungen besitzen, kondensiert.

11. Verfahren zur Herstellung der 5,6-Diaminopyrimidin-4(3H)-one der Formel 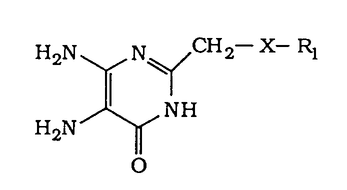 worin X und R₁ die in Anspruch 1 angegebenen Bedeutungen besitzen und die Zwischenprodukte für das Verfahren nach Anspruch 1 darstellen, **dadurch gekennzeichnet**, daß man ein Acetamidin der Formel 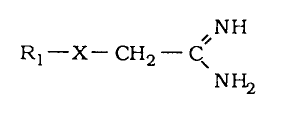 in der X und R₁ die oben angegebenen Bedeutungen besitzen. mit dem Iminoether des Cyanessigsäureethylesters der Formel 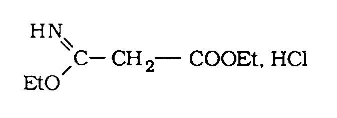 kondensiert zur Bildung eines 6-Aminopyrimidin-4(3H)-ons der Formel 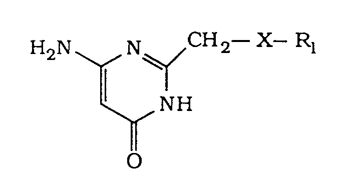 in der X und R₁ die oben angegebenen Bedeutungen besitzen, welches nitrosiert wird zur Bildung eines 6-Amino-5-nitrosopyrimidin-4(3H)-ons der Formel 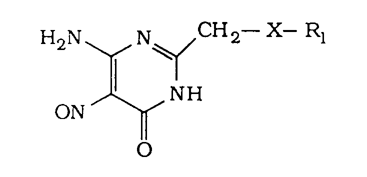 in der X und R₁ die oben angegebenen Bedeutungen besitzen, dessen Reduktion 5,6-Diaminopyrimidin-4(3H)-on liefert.

12. Verfahren zur Herstellung von pharmazeutischen Zubereitungen, **dadurch gekennzeichnet**, daß man mindestens eine Verbindung nach Anspruch 1 mit einem pharmazeutisch annehmbaren Trägermaterial vermischt.

13. Verfahren nach Anspruch 12, **dadurch gekennzeichnet**, daß das Trägermaterial für die Herstellung von Zubereitungen in Form von Aerosolen, Tabletten. dragierten Tabletten, Gelkapseln, injizierbaren Lösungen, Salben, Pudern, Lösungen oder Suppositorien geeignet ist.

## Patentansprüche (Patentansprüche für folgende(n) Vertragsstaat(en): GR)

1. Pteridin-4(3H)-one der Formel 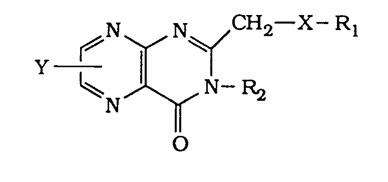 in der X ein Sauerstoffatom oder ein Schwefelatom,
Y ein Wasserstoffatom, eine C₁-C₄-Alkylgruppe, insbesondere eine Methylgruppe, in der 6-Stellung oder eine Hydroxylgruppe in der 7-Stellung,
R₁ ein Wasserstoffatom, eine C₁-C₄-Alkylgruppe, eine Phenylgruppe, die gegebe
nenfalls durch eine bis drei Gruppen ausgewählt aus C₁-C₄-Alkylgruppen, C₁-C₄-Alkoxygruppen, Halogenatomen, Hydroxylgruppen und Acetylgruppen substituiert ist, eine Benzylgruppe, die Methoxymethylgruppe, die Acetylgruppe, die 2-Acetoxyethylgruppe oder die 2,2,2-Trifluorethylgruppe und
R₂ ein Wasserstoffatom oder eine C₁-C₄-Alkylgruppe, insbesondere eine Methylgruppe, bedeuten;
und deren pharmazeutisch annehmbare Alkalimetallsalze.

2. Pteridin-4(3H)-one nach Anspruch 1, **dadurch gekennzeichnet**, daß X ein Sauerstoffatom, Y ein Wasserstoffatom oder eine Hydroxylgruppe in der 7-Stellung und R₂ ein Wasserstoffatom bedeuten; und deren pharmazeutisch annehmbare Alkalimetallsalze.

3. Pteridin-4(3H)-one nach Anspruch 1, **dadurch gekennzeichnet**, daß R₁ eine C₁-C₄-Alkylgruppe, die Phenylgruppe oder die Benzylgruppe bedeutet; und deren pharmazeutisch annehmbare Alkalimetallsalze.

4. Pteridin-4(3H)-on nach Anspruch 1, nämlich 2-(Methoxymethyl)-pteridin-4(3H)-on, und dessen pharmazeutisch annehmbare Alkalimetallsalze.

5. Pteridin-4(3H)-on nach Anspruch 1, nämlich 2-(Ethoxymethyl)-pteridin-4(3H)-on, und dessen pharmazeutisch annehmbare Alkalimetallsalze.

6. Pteridin-4(3H)-on nach Anspruch 1, nämlich 2-(Propoxymethyl)-pteridin-4(3H)-on, und dessen pharmazeutisch annehmbare Alkalimetallsalze.

7. Pteridin-4(3H)-on nach Anspruch 1, nämlich 2-(Phenoxymethyl)-pteridin-4(3H)-on, und dessen pharmazeutisch annehmbare Alkalimetallsalze.

8. Pteridin-4(3H)-on nach Anspruch 1, nämlich 2-(Benzyloxymethyl)-pteridin-4(3H)-on, und dessen pharmazeutisch annehmbare Alkalimetallsalze.

9. Pteridin-4(3H)-on nach Anspruch 1, nämlich 2-(Ethoxymethyl)-7-hydroxypteridin-4(3H)-on, und dessen pharmazeutisch annehmbare Alkalimetallsalze.

10. Verfahren zur Herstellung der Verbindungen nach Anspruch 1, **dadurch gekennzeichnet**, daß man ein 3-Aminopyrazin-2-carboxamid der Formel 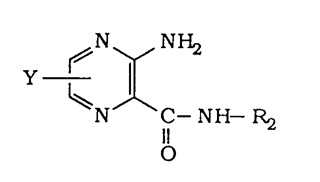 mit einem Orthoester der Formel R₁XCH₂C(OR₃)₃ umsetzt, worin X, Y, R₁ und R₂ die in Anspruch 1 angegebenen Bedeutungen besitzen und R₃ eine Alkylgruppe darstellt.

11. Verfahren zur Herstellung der Verbindungen nach Anspruch 1, worin R₂ ein Wasserstoffatom bedeutet, **dadurch gekennzeichnet**, daß man ein 4-Aminpteridin der Formel 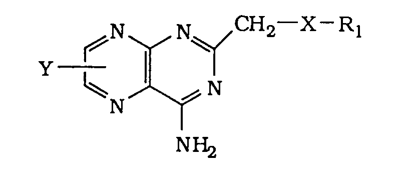 worin X, Y und R₁ die in Anspruch 1 angegebenen Bedeutungen besitzen, in alkalischem Wasser hydrolysiert.

12. Verfahren zur Herstellung der Verbindungen nach Anspruch 1, worin Y und R₂ Wasserstoffatome darstellen, **dadurch gekennzeichnet**, daß man ein 5,6-Diaminopyrimidin-4(3H)-on der Formel 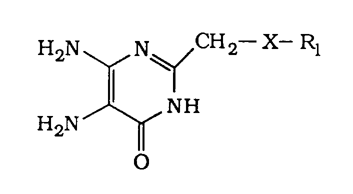 in der X und R₁ die in Anspruch 1 angegebenen Bedeutungen besitzen, mit Glyoxal behandelt.

13. Zwischenprodukte für die Herstellung der Verbindunge nach Anspruch 11, **gekennzeichnet durch** die Formel 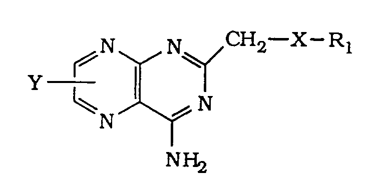 in der X, Y und R₁ die in Anspruch 1 angegebenen Bedeutungen besitzen.

14. Zwischenprodukte für die Herstellung der Verbindungen nach Anspruch 12, **gekennzeichnet durch** die Formel 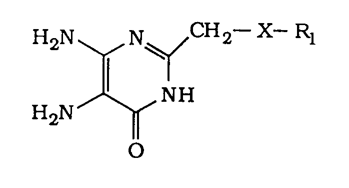 worin X und R₁ die in Anspruch 1 angegebenen Bedeutungen besitzen.

15. Verfahren zur Herstellung von pharmazeutischen Zubereitungen, **dadurch gekennzeichnet**, daß man mindestens eine Verbindung nach Anspruch 1 mit einem pharmazeutisch annehmbaren Trägermaterial vermischt.

16. Verfahren nach Anspruch 15, **dadurch gekennzeichnet**, daß das Trägermaterial für die Herstellung von Zubereitungen in Form von Aerosolen, Tabletten, dragierten Tabletten, Gelkapseln, injizierbaren Lösungen, Salben, Pudern, Lösungen oder Suppositorien geeignet ist.
